# EUROPÄISCHE PATENTSCHRIFT

(11) **EP 2 331 177 B1**
(45) Veröffentlichungstag und Bekanntmachung des Hinweises auf die Patenterteilung: **18.12.2013**
(21) Anmeldenummer: 09765236.6
(22) Anmeldetag: 16.06.2009
(51) Int. Cl.: A61M 5/32

(54) **INJEKTIONSVORRICHTUNG**
INJECTION DEVICE
DISPOSITIF D'INJECTION

(30) Priorität: 16.06.2008 AT 9582008; 04.06.2009 US 184044 P
(43) Veröffentlichungstag der Anmeldung: 15.06.2011
(73) Patentinhaber: Pharma Consult Ges.m.b.H. & Co Nfg KG, 1210 Wien (AT)
(72) Erfinder: SCHWIRTZ, Andreas, A-1090 Wien (AT); CSENAR, Markus, A-1050 Wien (AT)
(74) Vertreter: Burger, Hannes
(86) Internationale Anmeldenummer: PCT/AT2009/000239
(87) Internationale Veröffentlichungsnummer: WO 2009/152542

(56) Entgegenhaltungen:
- WO-A-2005/035029
- WO-A-2005/044345
- WO-A-2007/026163
- DE-C1- 19 925 904

## Beschreibung

Die Erfindung betrifft eine Injektionsvorrichtung, insbesondere einen Autoinjektor, zur Abgabe eines Medikaments, wie dieser im Anspruch 1 beschrieben ist.

Aus der WO 2005/044345 (nächstliegender Stand der Technik) A1 ist eine Autoinjektorvorrichtung bekannt geworden, welche an ihrer Außenseite ein Ampullengehäuse aufweist, innerhalb welcher eine Auslösehülse in Axialrichtung verschiebbar gelagert ist. Dabei ist die innere Auslösehülse in radialer Richtung gesehen über deren größten Teil der Gesamtlänge anliegend am äußeren Ampullengehäuse ausgebildet. Innerhalb der Auslösehülse ist die Ampulle mit einer daran aufgeschraubten Nadel sowie den dazu zugehörigen Antriebsfedern angeordnet. Der Nadelschutz für die Nadel ist innerhalb der Auslösehülse angeordnet und relativ zu dieser in Axialrichtung verschiebbar. Die Auslösehülse ist in deren Ausgangszustand mit ihrem vorderen Ende in etwa bündig mit dem vorderen Ende des Nadelschutzes ausgerichtet. Dabei ist die Nadel noch in einer geschützten Stellung innerhalb der Injektionsvorrichtung angeordnet. Beim Aufsetzen der Injektionsvorrichtung auf die Injektionsstelle wird die Auslösehülse in das Ampullengehäuse hinein geschoben und bewirkt dadurch weiters eine Verschiebung des Koppelelementes in Axialrichtung hin auf das hintere Ende des Ampullengehäuses, wodurch der Auslöseknopf mit dem Nadelsicherungselement so gekoppelt wird, dass bei einem gesonderten Eindrücken des Auslöseknopfes das Nadelsicherungselement aus einer den Ampullenträger sichernden Position herausgeschoben wird und der Ampullenträger durch die Nadelausschubfeder in Ausschubrichtung der Injektionsnadel gedrückt wird. Die Nadelausschubfeder ist einerseits am Ampullenträger und andererseits am Ampullengehäuse oder der Ampullenträgerzuführung abgestützt. Durch die Nadelausschubfeder wird die Nadel zusammen mit der Ampulle aus der Injektionsvorrichtung ausgefahren, wodurch der Einstich im Bereich der vorgesehenen Einstichstelle erfolgt. Hierdurch wird gleichzeitig der Spritzenhalter in eine vordere Position geschoben, wodurch die zwischen dem Spritzenhalter und dem Nadelschutz angeordnete Nadelschutzfeder gespannt bzw. komprimiert wird. Durch die Nadelausschubfeder wird der Ampullenträger in der Ampullenträgerführung soweit nach vorne geschoben, dass Verriegungselemente den an der Innenseite der Ampullenträgerführung vorgesehenen Ausnehmung gegenüberliegen. In dieser Stellung werden die radial nach außen vorgespannten Verriegelungselemente in diese Ausnehmungen hinein gedrückt, wodurch der Stopfenhalter entsichert und von dem Ampullenträger gelöst wird. Die zwischen dem hinteren Ende des Ampullenträgers und dem Stößel gespannte Injektionsfeder kann sich dann entspannen, wodurch der Stopfen in die Ampulle eingeschoben wird. Dadurch wird die in der Ampulle enthaltene Substanz verdrängt und durch die ausgefahrene Nadel abgegeben. Wird die gesamte Injektionsvorrichtung nach der Durchführung der Injektion von der Injektionsstelle abgenommen, so wird der durch die gespannte Nadelschutzfeder in Richtung der Injektionsnadel vorgespannte Nadelschutz nach vorne aus der Injektionsvonichtung heraus über die Nadel geschoben. Bei dieser Ausführungsform ist die Auslösehülse innerhalb des Ampullengehäuses angeordnet, wobei die tatsächliche Auslösung und Aktivierung durch den quer zur Längsachse zu betätigenden Auslöseknopf erfolgt.

Die WO 2005/113039 A1 beschreibt einen Autoinjektor zur Abgabe eines Medikaments, der ein feststehendes Traggehäuse mit einer inneren und äußeren Gehäusewand im Bereich des distalen Endes umfasst. Im Traggehäuse ist die Antriebsbaugruppe, der Speicherbehälter für das Medikament sowie die Nadelanordnung angeordnet. Das proximale Ende der Nadel steht mit dem Innenraum des Speicherbehälters und somit mit dem Medikament stets in Strömungsverbindung. Weiters umfasst die automatische Injektionsvorrichtung ein in Richtung der Längsachse verschiebbares Nadelschutzelement, welches zwischen der inneren und äußeren Gehäusewand des Traggehäuses geführt gelagert ist. Diesem hülsenförmigen Nadelschutzelement ist eine weitere Antriebsbaugruppe zugeordnet, um dieses nach der Freigabe in die abdeckende Stellung der Nadel zu verbringen. Zusätzlich kann an der Außenseite des Traggehäuses eine die Handhabbarkeit verbessernde Grifflage angeordnet sein. An dem der Nadel gegenüberliegenden Ende - also dem proximalen Ende - ist eine Auslöseeinrichtung zur Auslösung der ersten Antriebsbaugruppe angeordnet. Dabei erfolgt die Auslösebewegung durch eine Schiebebewegung in senkrechter Richtung bezüglich der Längsachse. Nach erfolgter Querverlagerung und der Entriegelung der Rückhaltevorrichtung wird die erste Antriebsbaugruppe aktiviert, wodurch der Kolben mitsamt dem Speicherbehälter soweit in Richtung des distalen Endes verlagert, bis dass ein dem proximalen Ende zugewendeter Anschlag des Speicherbehälters an einem Anschlag der inneren Gehäusewand des Traggehäuses zur Anlage kommt. Gleichzeitig mit dieser Längsverstellung erfolgt auch die Auslösung des Nadelschutzelements, welches dadurch bis hin zur Anlage an der Abgabestelle des Medikaments bewegt wird. Nach erfolgter Abgabe des Medikaments und dem Abziehen der gesamten Injektionsvorrichtung erfolgt aufgrund der bereits aktivierten weiteren Antriebsvorrichtung eine weitere Vorwärtsbewegung des Nadelschutzelements, bis dass die Nadel vollständig abgedeckt ist. Aus der US 6,767,336 B 1 ist eine automatisierte Injektionsvorrichtung zur Abgabe eines Medikaments bekannt geworden, die ein Traggehäuse umfasst, in dem die Antriebsbaugruppe, der Speicherbehälter für das Medikament sowie die Nadelanordnung angeordnet ist. Über die Kanüle der Nadelanordnung ist ein elastisch verformbares und durchstechbares Schutzelement übergestülpt, welches mit dem Nadelhalter verbunden ist. Der Nadelhalter trägt einerseits die Nadel und ist am Speicherbehälter für das Medikament an seinem distalen Ende angekuppelt. Die Kanüle steht dabei mit dem Innenraum und somit mit dem Medikament stets in Strömungsverbindung. Im Bereich des distalen Endes ist am Traggehäuse ein Nadelschutzelement längs verschieblich gelagert, welchem ein weiteres Antriebsmittel zugeordnet ist. Ausgehend vom proximalen Ende des Traggehäuses ist diesem eine Auslösehülse zugeordnet bzw. nimmt diese das Traggehäuse zumindest bereichsweise auf. Der ersten Antriebsbaugruppe ist weiters ein Sicherungselement zugeordnet, um eine ungewollte Auslösung zu vermeiden. Die Auslösung des Nadelschutzelements bzw. dessen Antriebsmittel erfolgt während der relativen Verlagerung des Medikamentenspeichers mit samt der daran angeordneten Nadelanordnung. Sowohl die Auslösehülse als auch das Nadelschutzelement sind jeweils eng anliegend sowie in axialer Richtung hintereinander am gemeinsamen Traggehäuse angeordnet.

Die WO 2005/113039 A1 beschreibt einen Autoinjektor zur Abgabe eines Medikaments, der ein feststehendes Traggehäuse mit einer inneren und äußeren Gehäusewand im Bereich des distalen Endes umfasst. Im Traggehäuse ist die Antriebsbaugruppe, der Speicherbehälter für das Medikament sowie die Nadelanordnung angeordnet. Das proximale Ende der Nadel steht mit dem Innenraum des Speicherbehälters und somit mit dem Medikament stets in Strömungsverbindung. Weiters umfasst die automatische Injektionsvorrichtung ein in Richtung der Längsachse verschiebbares Nadelschutzelement, welches zwischen der inneren und äußeren Gehäusewand des Traggehäuses geführt gelagert ist. Diesem hülsenförmigen Nadelschutzelement ist eine weitere Antriebsbaugruppe zugeordnet, um dieses nach der Freigabe in die abdeckende Stellung der Nadel zu verbringen. Zusätzlich kann an der Außenseite des Traggehäuses eine die Handhabbarkeit verbessernde Grifflage angeordnet sein. An dem der Nadel gegenüberliegenden Ende - also dem proximalen Ende - ist eine Auslöseeinrichtung zur Auslösung der ersten Antriebsbaugruppe angeordnet. Dabei erfolgt die Auslösebewegung durch eine Schiebebewegung in senkrechter Richtung bezüglich der Längsachse. Nach erfolgter Querverlagerung und der Entriegelung der Rückhaltevorrichtung wird die erste Antriebsbaugruppe aktiviert, wodurch der Kolben mitsamt dem Speicherbehälter soweit in Richtung des distalen Endes verlagert, bis dass ein dem proximalen Ende zugewendeter Anschlag des Speicherbehälters an einem Anschlag der inneren Gehäusewand des Traggehäuses zur Anlage kommt. Gleichzeitig mit dieser Längsverstellung erfolgt auch die Auslösung des Nadelschutzelements, welches dadurch bis hin zur Anlage an der Abgabestelle des Medikaments bewegt wird. Nach erfolgter Abgabe des Medikaments und dem Abziehen der gesamten Injektionsvorrichtung erfolgt aufgrund der bereits aktivierten weiteren Antriebsvorrichtung eine weitere Vorwärtsbewegung des Nadelschutzelements, bis dass die Nadel vollständig abgedeckt ist.

Aus der EP 0 307 367 B 1 bzw. der DE 38 72 122 T2 ist eine Injektionsvorrichtung zur Abgabe eines Medikaments bekannt geworden, bei der das Medikament durch Aufziehen im Spritzenzylinder aufgenommen werden kann. Eine Sicherungsvorrichtung für einen an der äußeren Seite des Spritzenzylinders angeordneten Nadelschutz wird mittels eines kegelig ausgebildeten Teils an der Kolbenstange aktiviert. Beim Ausspritzen bzw. bei der Abgabe des Medikaments wird die Sicherungsvorrichtung durch den kegeligen Teil ausgelöst, wobei unter Beaufschlagung einer Federkraft die außen liegende Nadelschutzhülse entlang des Spritzenzylinders verstellt wird, bis die über den Spritzenzylinder vorragende Nadel abgedeckt ist.

Aus der US 5,295,965 A ist eine automatisierte Injektionsvorrichtung zur Abgabe eines Medikaments bekannt geworden, bei welcher die äußere Umhüllung eine Nadelschutzhülse darstellt. Diese reicht von dem dem Patienten zugewendeten distalen Ende bis nahe an das proximale Ende hin und kann dort mit einer kappenförmigen Auslösemuffe betätigt werden. Für die Aktivierung ist ein im Bereich des proximalen Endes angeordneter Sicherungsstift zu lösen, der die erste Antriebsbaugruppe bis zur Auslösung mit der kappenförmigen Auslösemuffe sichert. Nach dem Entfernen des Sicherungsstifts, kann die muffenförmige Auslösemuffe relativ gegenüber dem Traggehäuse sowie der Nadelschutzhülse bewegt werden, wodurch sowohl die erste Antriebsbaugruppe als auch die Nadelschutzhülse ausgelöst wird. Der Nadelschutzhülse ist ihrerseits eine eigene Federvorrichtung zugeordnet, mit welcher dieser in die die Nadel abdeckende Stellung verstellt wird.

Aus der US 5,658,259 A bzw. der EP 0 956 058 B1 ist eine weitere automatisierte Injektionsvorrichtung zur Abgabe eines Medikaments aus einer Karpule bekannt geworden. Dazu wird innerhalb eines Traggehäuses die Karpule mit dem Medikament angeordnet, wobei dieser zusätzlich noch eine erste Antriebsbaugruppe für deren Betätigung und die Abgabe des Medikaments zugeordnet ist. Innerhalb des Traggehäuses ist eine durch die Längsverstellung des Nadelträgers auslösbare Nadelschutzhülse angeordnet, welcher ebenfalls ein Verstellelement zugeordnet ist. Die Nadelschutzhülse wird innerhalb des Tragkörpers mittels einer Rastverbindung bis zu deren Auslösung durch den verstellten Nadelträger gehalten. Nach der Abgabe des Medikaments deckt die Nadelschutzhülse das über den Autoinjektor vorragende Ende der Nadel ab.

Die US 4,031,893 A beschreibt einen weiteren Autoinjektor, bei welchem in einem gemeinsamen Traggehäuse die auslösbare Antriebsbaugruppe und der Medikamentenbehälter mit der daran angeordneten Nadelanordnung aufgenommen ist. Die Antriebsbaugruppe ist wiederum durch ein Sicherungselement gegen unbeabsichtigtes Auslösen gesichert. Das Traggehäuse ist an seiner Außenseite von einem hülsenförmigen Bauteil umgeben, welcher an seinem proximalen Ende mit einer zusätzlichen Auslösehülse gekuppelt ist. Die Auslösehülse bewirkt nach dem Entfernen der Sicherungskappe mitsamt dem Sicherungsstift die Auslösung der Antriebsbaugruppe, wodurch der Medikamentenbehälter mitsamt der Nadelanordnung in Richtung des distalen Endes verstellt wird. Die mit dem Innenraum des Medikamentenbehälters stets in Strömungsverbindung stehenden Kanüle ist von einer elastisch verformbaren Schutzhülle umgeben, welche an der Innenseite des distalen Endes des Traggehäuses abgestützt ist. Während der Vorwärtsbewegung durchsticht die Kanüle die elastisch verformbare Schutzhülle und tritt aus dem Traggehäuse zur Injektion aus. Die elastisch verformbare Nadelschutzhülle bewirkt nach Beenden des Injektionsvorganges eine Rückstellung der Nadelanordnung mitsamt dem Medikamentenbehälter in den Innenraum des Traggehäuses.

Der vorliegenden Erfindung liegt die Aufgabe zugrunde, eine Injektionsvorrichtung, insbesondere einen Autoinjektor zu schaffen, welcher bei einfacher Bedienung eine hohe Betriebssicherheit, insbesondere einen verbesserten Nadelschutz bietet. Darüber hinaus soll auch noch ein Verfahren zur Sterilisation angegeben werden, bei welchem zumindest die gesamte Nadelanordnung auch während einer längeren Aufbewahrungsdauer gesichert sterilisiert bleibt.

Diese Aufgabe der Erfindung wird dadurch gelöst, dass das Nadelschutzelement mit der dieser zusammenwirkenden zweiten Antriebsbaugruppe im Axialschnitt gesehen radial zwischen dem Traggehäuse und der Aktivierungshülse angeordnet ist und dass das Traggehäuse einen vorderen und einen hinteren Traggehäuseteil mit jeweils einem vorderen und hinteren Aufnahmeraum umfasst, und die beiden Traggehäuseteile in einem Verbindungsabschnitt über eine Kupplungsvorrichtung in Richtung der Längsachse in zwei unterschiedlichen Längspositionen in einer ersten und einer zweiten Kupplungsstellung miteinander kuppelbar sind.

Der sich durch die Merkmale des Anspruches 1 ergebende überraschende Vorteil liegt darin, dass die Aktivierungshülse auch noch die dem Nadelschutzelement zugeordnete Antriebsbaugruppe in sich aufnimmt, wodurch Manipulationen für eine mögliche nachträgliche Verwendung gesichert vermieden werden. Weiters wird dadurch aber auch über die gesamte Lagerung in der Aufbewahrungsstellung eine Manipulation an den Antriebsbaugruppen sowie dem Nadelschutzelement vermieden. Weiters wird aber auch noch ein zusätzlicher Schutz der gesamten innen liegenden Bauteile durch die nahezu vollständige Abdeckung durch die Aktivierungshülse erzielt. Durch die einfache Bedienung sind weiters auch Fehlbedienungen praktisch ausgeschlossen und es sind keine zusätzlichen Verstellbewegungen bzw. Auslösebewegungen notwendig, um die Abgabe des Medikaments einzuleiten. Ein weiterer Vorteil liegt noch darin, dass die Aktivierungs- bzw. Betätigungshülse nach deren Freigabe durch die Sicherungsvorrichtung einfach durch den Benutzer mit einer Einhandbetätigung gleichzeitig mit der gesamten Injektionsvorrichtung an die für die Abgabe vorgesehene Körperstelle heranzuführen ist und durch entsprechende Abstützung des Traggehäuses der Injektionsvorrichtung an dieser Körperstelle und einer entsprechender Druckbeaufschlagung eine relative Verlagerung der Aktivierungshülse relativ gegenüber dem Traggehäuse erzielt werden kann. Dadurch wird der Abgabevorgang des Medikaments eingeleitet und durchgeführt. Somit ist die gesamte Injektionsvorrichtung lediglich durch das Entfernen der Sicherungskappe der Sicherungsvorrichtung für den nachfolgenden Abgabevorgang vorzubereiten und mit einer weiteren einzigen Druckbewegung eingeleitet über die Aktivierungshülse auf die entsprechende Körperstelle das Medikament automatisiert zu verabreichen. Darüber hinaus ist es auch noch vorteilhaft, da durch die Trennung des Traggehäuses in zwei zueinander verschiebbare Traggehäuseteile unterschiedliche Betriebszustände erreicht werden können, welche im Zuge der Sterilisierung und der nachfolgenden Lagerdauer von bedeutendem Einfluss sind.

Vorteilhaft ist weiters eine Ausbildung nach Anspruch 2, da so eine zusätzliche Führung zwischen den zu kuppelnden Traggehäuseteilen geschaffen wird, wobei gleichzeitig noch in diesem Bereich vordefinierte Kupplungspositionen geschaffen werden können.

Durch eine weitere Ausbildung ist es möglich, in der ersten Kupplungsstellung bereits eine eindeutige Lagepositionierung der beiden Traggehäuseteile zueinander zu erzielen und zusätzlich die Möglichkeit für einen in dieser Stellung externen Zugang in den vorderen Traggehäuseteil zu schaffen.

Nach einer anderen Ausführungsvariante gemäß Anspruch 3 wird lediglich eine Bewegungsrichtung der beiden Traggehäuseteile aufeinander zu ermöglicht, jedoch ein ungewolltes Trennen, wie beispielsweise durch Erschütterungen und Schlagbeanspruchungen usw., gesichert verhindert.

Vorteilhaft ist auch eine Weiterbildung nach Anspruch 4, da so das Traggehäuse in seinem distalen Ende auch ein Durchstechen durch den Dichtstopfen ermöglicht, jedoch durch diesen ein steriler Abschluss des vorderen Aufnahmeraums in Verbindung mit dem vorderen Traggehäuseteil während der gesamten Aufbewahrungsdauer sichergestellt ist.

Bei der Ausgestaltung nach Anspruch 5 ist von Vorteil, dass so an einer vorbestimmten Stelle ein für die später stattfindende Sterilisation notwendiger Zugang in den vorderen Aufnahmeraum geschaffen werden kann, um die darin enthaltene bzw. aufgenommene Nadelanordnung auch bei bereits vormontierter Injektionsvorrichtung sterilisieren zu können.

Durch die Weiterbildung nach Anspruch 6 wird erreicht, dass so ein vordefinierter Anschlagbereich einerseits für das Dichtelement und andererseits für die Abstützung der zweiten Antriebsbaugruppe geschaffen werden kann.

Durch die Ausbildung nach Anspruch 7 kann nach der durchgeführten Sterilisierung eine eindeutige und sichere Abdichtung des sterilisierten vorderen Aufnahmeraums erfolgen. Darüber hinaus kann das umlaufende Dichtelement auch als dämpfende Halterung für die im Traggehäuse aufgenommene Karpule mit dem Medikament dienen. Dadurch können Stöße und Schlagbeanspruchungen, ausgehend von der äußeren Aktivierungshülse hin zur Karpule mit dem Medikament gedämpft bzw. vollständig reduziert bzw. abgefangen werden.

Vorteilhaft ist auch eine Ausbildung nach Anspruch 8, da so für die Begasung im Zuge der ETO-Sterilisierung nur ein vordefinierter Innenraum der Sterilisation zu unterziehen ist. Gemäß einer Ausbildung wie im Anspruch 9 beschrieben, da so bei bereits vormontierter Injektionsvorrichtung trotzdem zwischen den einzelnen Bauteilen hindurch ein Zugang in den vorderen Aufnahmeraum für die nachträglich durchzuführende Sterilisation geschaffen werden kann.

Dabei erweist sich eine Ausgestaltung nach Anspruch 10 vorteilhaft, da so mit kurzem Verstellweg zwischen den beiden Traggehäuseteilen nach der Sterilisation eine einwandfreie Abdichtung auch über eine längere Lagerdauer gesehen, erzielbar ist.

Nach einer vorteilhaften Weiterbildung gemäß Anspruch 11 wird zwischen der Aktivierungshülse und dem Traggehäuse lediglich ein Ineinander- bzw. Übereinanderschieben ermöglicht, jedoch eine ungewollte Trennung der beiden Teile voneinander gesichert verhindert. Dadurch wird eine sehr hohe Betriebssicherheit erzielt und Manipulationen nahezu ausgeschaltet.

Gemäß Anspruch 12 wird so während der Aufbewahrungsstellung eine eindeutige Lagefixierung zwischen dem Nadelschutzelement und dem Traggehäuse erzielt. Diese Arretierung kann durch eine einfache Schwenkbewegung des Hebelelements gelöst werden, wobei hiezu eine relative Längsverstellung zwischen dem Traggehäuse und der Aktivierungshülse erforderlich ist, wobei im Zusammenwirken mit der an der Aktivierungshülse ausgebildeten Anschlagfläche die Verstellung des rippenartigen Hebelelements und damit die Freigabe des Nadelschutzelements erfolgt.

Von Vorteil ist aber auch eine Ausbildung nach Anspruch 13, da so eine eindeutig vorbestimmbare Steuerfläche ausgebildet wird und nach einem vorbestimmbaren relativen Verstellweg zwischen der Aktivierungshülse und dem Traggehäuse auch eine Auslösung bzw. Aktivierung des Nadelschutzelements erfolgt. Dieses wird mittels der mit dieser zusammenwirkenden zweiten Antriebsbaugruppe in eine die Kanüle nach dem Gebrauch abdeckende Stellung verstellt. Weiters wird bei dieser Ausbildung eine erneute Rückstellung und damit verbunden eine erneute Freigabe der bereits benutzten Nadelspitze der Kanüle verhindert.

Möglich ist dabei auch eine Ausbildung nach Anspruch 14, da so eine zusätzliche mechanische Fixierung des Nadelschutzelements zwischen dem Traggehäuse und der Aktivierungshülse erfolgt. Weiters wird durch diese Ausgestaltung eine Anschlagbegrenzung für die Vorwärtsbewegung des Nadelschutzelements in die abdeckende Stellung ermöglicht. Dadurch können die Stellkräfte des Antriebsmittels der zweiten Antriebsbaugruppe etwas höher ausgewählt werden, um so eine einwandfreie abdeckende Stellung in jedem Fall gesichert zu erzielen.

Vorteilhaft ist die Ausbildung nach Anspruch 15, da so eine vorgefertigte Baugruppe geschaffen werden kann, welche an der Karpule angeordnet bzw. daran gehaltert werden kann. Diese gesamte Nadelanordnung ist so ausgelegt, dass die der Karpule bzw. dem Karpulenverschluss zugewendete Nadelspitze während der gesamten Aufbewahrungsstellung den Karpulenverschluss noch nicht durchstochen hat und somit kein in der Karpule bevorratetes Medikament austreten kann. Die Strömungsverbindung zwischen der Kanüle und dem Innenraum der Karpule wird erst während dem Injektionsvorgang hergestellt.

Von Vorteil ist aber auch eine Ausbildung nach Anspruch 16, da so der Nadelträger in einer eindeutigen festgelegten Positionierung gegenüber dem Führungselement gehalten werden kann und erst nach einem vorbestimmbaren Verstellweg der Karpule relativ gegenüber dem Traggehäuse die Strömungsverbindung zwischen der Kanüle und dem Innenraum der Karpule hergestellt wird. Weiters ist es auch möglich, dass im Zusammenwirken mit einer Steuerkurve erst an einer vorbestimmbaren Stelle bzw. nach einem vorbestimmbaren Verstellweg der Einstichvorgang des Nadelendes durch den Karpulenverschluss hindurch erfolgt und so ein Zugang zum abzugebenden Medikament geschaffen wird.

Vorteilhaft ist die Ausbildung nach Anspruch 17, da so durch die Reduktion der Abmessung im Bereich der Kolbenstange eine größere Distanzierung von der Innwand der Karpule erreicht wird und so bei einer Schlagbeanspruchung eine Beschädigung der Karpule verhindert werden kann.

Vorteilhaft ist auch eine weitere Ausführungsform nach Anspruch 18, da so mit einem eindeutigen kurzen Verstellweg zwischen der Aktivierungshülse und dem Traggehäuse der Injektionsvorgang ausgelöst werden kann, welcher nach Auslösung bzw. Aktivierung der Antriebsbaugruppe automatisiert durchgeführt wird.

Vorteilhaft ist weiters eine Ausbildung nach Anspruch 19, da so während der relativen Längsverstellung der gesamten Nadelbaugruppe mit der Karpule einerseits der Zeitpunkt des Einstichs in den Patenten und andererseits die Herstellung der Strömungsverbindung mit dem Innenraum der Karpule festgelegt werden kann.

Durch die Ausbildung nach Anspruch 20 ist es möglich, zuerst einen gesicherten Einstich von der Kanüle in den Patienten zu erzielen und anschließend erst den Zugang zum Medikament herzustellen. Dadurch wird vermieden, dass noch vor dem Einstich der Kanüle in den Patienten das Medikament aus der Nadel ungewollt austritt.

Nach einer anderen Ausführungsvariante gemäß Anspruch 21 wird eine hohe Betriebssicherheit der gesamten Injektionsvorrichtung erreicht, da bereits mit der Aktivierung der ersten Antriebsbaugruppe auch der Nadelschutz für die abdeckende Stellung der benützten Kanüle aktiviert wird.

Vorteilhaft ist auch eine Weiterbildung nach Anspruch 22, da so mit einer einzigen Verstellbewegung eine gleichzeitige Auslösung erfolgt und dadurch bei einer möglichen Fehlfunktion bereits vor der Abgabe des Medikaments auch bereits eine Abdeckung der das Traggehäuse überragenden Nadelspitze gesichert erfolgt.

Weiters ist auch eine Ausbildung möglich, bei welcher mit einer schlanken Bauweise das Auslangen gefunden werden kann und zusätzlich eine relativ exakte Längsführung des Nadelschutzelements am Traggehäuse erzielbar ist.

Es ist aber auch eine Ausbildung, wie im Anspruch 23 beschrieben möglich, da so eine relative Anordnung der Haltearme bezüglich der Haltescheibe erfolgt und dadurch ein gegenseitiges Verklemmen gesichert verhindert wird. Weiters kann so eine noch sicherere Wirkung der gesamten Sicherungsvorrichtung für die erste Antriebsbaugruppe erzielt werden.

Vorteilhaft ist auch eine Ausbildung gemäß Anspruch 24, weil so eine von der Montage externe Sterilisation ermöglicht wird, ohne dass dadurch nach der Sterilisation erneut Verunreinigungen in den noch nicht vollständig abgeschlossenen sterilisierten Aufnahmeraum um die Nadelanordnung gelangen können.

Von Vorteilhaft ist auch eine Weiterbildung nach Anspruch 25, weil so auch mehrere derartiger Injektionsvorrichtungen nebeneinander geordnet aufbewahrt werden können. Darüber hinaus wird auch noch eine von der Montage externe Sterilisation ermöglicht, ohne dass dadurch nach der Sterilisation erneut Verunreinigungen in den noch nicht vollständig abgeschlossenen sterilisierten Aufnahmeraum um die Nadelanordnung gelangen können.

Zum besseren Verständnis der Erfindung wird diese anhand der nachfolgenden Figuren näher erläutert.

Es zeigen jeweils in stark schematisch vereinfachter Darstellung:
- Fig. 1: eine erfingdungsgemäß ausgebildete Injektionsvorrichtung vor dem Sterilisieren des Aufnahmeraums mit der darin angeordneten Nadelanordnung, im Axialschnitt und vereinfachter Darstellung;
- Fig. 2: einen Teilbereich der Injektionsvorrichtung nach Fig. 1, jedoch in einer dazu anderen Schnittebene;
- Fig. 3: die Injektionsvorrichtung nach den Fig. 1 und 2 in bereits sterilisiertem Zustand, im Axialschnitt und vereinfachter Darstellung;
- Fig. 4: die Injektionsvorrichtung nach den Fig. 1 bis 3, während dem Abgabevorgang des Medikaments, im Axialschnitt und vereinfachter Darstellung;
- Fig. 5: die Injektionsvorrichtung nach den Fig. 1 bis 4 bei einer die Kanüle abdeckenden Stellung des Nadelschutzelements, im Axialschnitt und vereinfachter Darstellung
- Fig. 6: die Injektionsvorrichtung nach Fig. 5, jedoch in einer dazu anderen Schnittebene;
- Fig. 7: die Haltescheibe der Sicherungsvorrichtung in Draufsicht und vereinfachter Darstellung;
- Fig. 8: die erfindungsgemäße Injektionsvorrichtung nach Fig. 1 vor bzw. während dem Sterilisieren in einer dafür vorgesehenen Aufnahmeschale, in Ansicht geschnitten nach den Linien VIII - VIII in Fig. 10;
- Fig. 9: die Injektionsvorrichtung nach Fig. 8 in der Aufnahmeschale in jener Kupplungsstellung nach dem Sterilisieren, in der vollständig abgedichteten Position;
- Fig. 10: einen Teilabschnitt der Aufnahmeschale nach den Fig. 8 und 9, in schaubildlich vereinfachter Darstellung, ohne Injektionsvorrichtung;
- Fig. 11: die Nadelanordnung in der gekuppelten Stellung an der Karpule nach den Fig. 1 bis 6, in schaubildlich vereinfachter Darstellung;
- Fig. 12: die Nadelanordnung nach Fig. 11 bei abgehobenem Führungsteil des Führungselements, in schaubildlich vereinfachter Darstellung;
- Fig. 13: die Nadelanordnung in der gekuppelten Stellung an der Karpule nach den Fig. 1 bis 6 während der Verstellbewegung zur Abgabe des Medikaments in einer Stellung unmittelbar vor dem Zusammenwirken der Stellnocke mit der Steuerkurve, im Axialschnitt;
- Fig. 14: eine erste vormontierte Baugruppe der Injektionsvorrichtung im Bereich des vorderen Traggehäuseteils, im Axialschnitt;
- Fig. 15: eine zweite vormontierte Baugruppe der Injektionsvorrichtung im Bereich des hinteren Traggehäuseteils, im Axialschnitt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

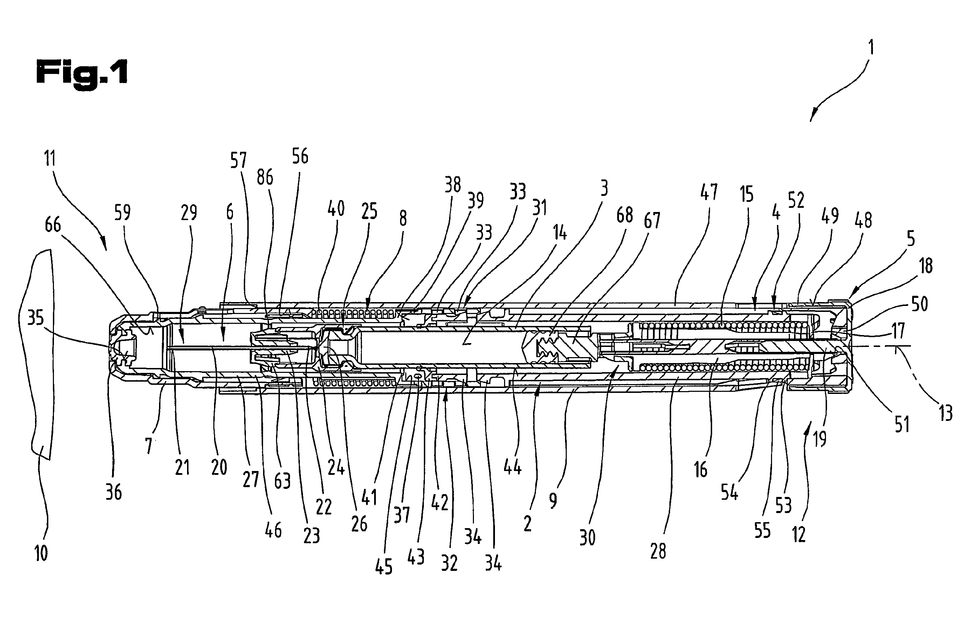

In den Fig. 1 bis 6 ist eine Injektionsvorrichtung 1, insbesondere ein Autoinjektor in verschiedenen Betriebsstellungen vereinfacht dargestellt. Bei derartigen Autoinjektoren, welche zur automatischen Verabreichung eines Medikamentes verwendet werden, wird eine so genannte Aufbewahrungsstellung und eine Injektionsstellung unterschieden, wobei zwischen diesen beiden Stellungen automatisierte Stell- bzw. Verlagerungsvorgänge ablaufen und damit verbunden die Abgabe des Medikaments erfolgt.

Die Injektionsvorrichtung 1 ist aus einer Vielzahl von Bauteilen zusammengesetzt und kann ein Traggehäuse 2, ein in diesem aufgenommenes Speichergefäß wie eine Karpule 3, eine erste auslösbare Antriebsbaugruppe 4, die mit der Karpule 3 in Wirkverbindung steht, eine Sicherungsvorrichtung 5, eine Nadelanordnung 6, ein Nadelschutzelement 7 sowie eine zweite, dem Nadelschutzelement 7 zugeordnete bzw. damit in Wirkverbindung stehende zweite Antriebsbaugruppe 8 sowie eine Aktivierungshülse 9 umfassen.

Die Injektionsvorrichtung 1 bzw. das Traggehäuse 2 weist ein einem Lebewesen wie einem Patienten 10 zuwendbares distales Ende 11 sowie ein davon abgewendetes proximales Ende 12 auf, wobei sich zwischen den beiden Enden 11, 12 eine Längsachse 13 erstreckt. Weiters wird die Bezeichnung für die Ortsangabe vorne und hinten verwendet und dabei vorne dem distalen Ende 11 und hinten dem proximalen Ende 12 zugeordnet. Das Aufnahmegefäß für ein Medikament 14 ist hier durch die Karpule 3 gebildet, in der das Medikament 14 bzw. der abzugebende und zu injizierende Wirkstoff bereits während der Aufbewahrungsstellung darin bevorratet und abgabefertig vorliegt. Die Karpule 3 ist dabei im Traggehäuse 2 aufgenommen bzw. von diesem umgeben. Bei der Karpule 3 handelt es sich um handelsübliche Verpackungen für das Medikament 14, wobei die darin aufgenommene Menge des Medikaments 14 je nach Anwendungsfall darauf abgestimmt werden kann. Weiters ist in bekannter Weise der Karpule 3 an dem dem proximalen Ende 12 der Injektionsvorrichtung 1 zugewendeten Ende die erste auslösbare Antriebsbaugruppe 4 zugeordnet bzw. steht diese damit in Wirkverbindung.

Die erste Antriebsbaugruppe 4 umfasst ein erstes Antriebsmittel 15, welches durch die Sicherungsvorrichtung 5 so lange gegen eine Freigabe gesichert ist, bis dass durch einen Benutzer bewusst eine Aktivierung der Injektionsvorrichtung 1 erfolgt, bei der die erste Antriebsbaugruppe 4 freigegeben und damit die Injektionsvorrichtung 1 ausgelöst wird. Durch die Sicherungsvorrichtung 5 ist somit die erste Antriebsbaugruppe 4 bis zu deren Aktivierung für den Injektionsvorgang bezüglich des Traggehäuses 2 in ihrer Position festgelegt. Die erste Antriebsbaugruppe 4, insbesondere das Antriebsmittel 15 und die dem Antriebsmittel 15 zugeordneten Haltearme 16 sind innerhalb des Traggehäuses 2 aufgenommen. Die Haltearme 16 greifen ihrerseits in eine im Bereich des proximalen Endes 12 der Injektionsvorrichtung 1 angeordnete Haltescheibe 17 ein, wobei die detaillierte Beschreibung derselben nachfolgend erfolgen wird. Gegen ein unbeabsichtigtes Auslösen der Antriebsbaugruppe 4 umfasst die Sicherungsvorrichtung 5 weiters eine Sicherungskappe 18, wobei in deren Zentrum ein die Haltescheibe 17 durchragender Sicherungsstift 19 angeordnet ist. Der Sicherungsstift arretiert in bekannter Weise die Haltearme 16 in radialer Richtung bezüglich der Längsachse 13 und verhindert bis zum Entfernen der Sicherungskappe 18 mitsamt dem Sicherungsstift 19 ein Loslösen der an der Haltescheibe 17 verrastet gehaltenen Haltearme 16 in radialer Richtung auf die Längsachse 13.

Im Bereich des distalen Endes 11 ist der Karpule 3 die Nadelanordnung 6 unmittelbar vorgeordnet. Die Nadelanordnung 6 umfasst ihrerseits eine Kanüle 20 bzw. eine hohl ausgebildete Nadel mit in Richtung der Längsachse 13 voneinander distanzierten Nadelenden 21, 22. Die Kanüle 20 wird ihrerseits von einem Nadelträger 23 gehalten bzw. ist diese darin aufgenommen. Beide Nadelenden 21, 22 überragen jeweils beidseits den Nadelträger 23.

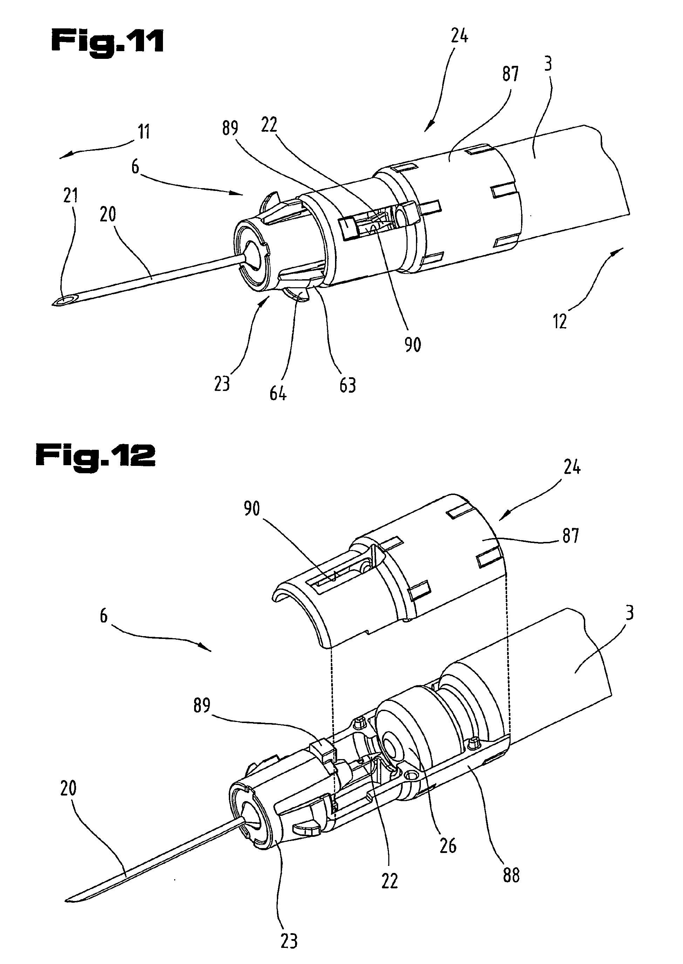

Der Nadelträger 23 ist seinerseits über ein Führungselement 24 an einem dem distalen Ende 11 zugewendeten Karpulenende 25 angekuppelt bzw. damit verbunden. Nähere Details siehe dazu auch noch in den nachfolgenden Fig. 11 und 12. Die Karpule 3 weist ihrerseits an dem dem distalen Ende 11 zugewendeten Karpulenende 25 einen durchstechbaren Karpulenverschluss 26 auf Das dem Karpulenverschluss 26 zugewendete proximale Nadelende 22 ist mittels des Nadelträgers 23 derart am Führungselement 24 angeordnet, dass in der Aufbewahrungsstellung der Injektionsvorrichtung 1 das Nadelende 22 noch distanziert vom Karpulenverschluss 26 angeordnet ist. Damit ist vorerst ein Zugang in den Innenraum der Karpule 3 hin zum Medikament 14 unterbunden. Weiters sind in der Aufbewahrungsstellung der Injektionsvorrichtung 1 noch beide Nadelenden 21, 22 innerhalb des Traggehäuses 2 angeordnet und somit Stichverletzungen unterbunden. Weiters kann, wie dies nachfolgend noch näher erläutert werden wird, so auch die gesamte Nadelanordnung 6 während der gesamten Aufbewahrungsdauer bis hin zum bestimmungsgemäßen Einsatz der Abgabe des Medikaments 14 in völlig sterilem Zustand gehalten werden.

Das mit der zweiten Antriebsbaugruppe 8 in Wirkverbindung stehende Nadelschutzelement 7 ist mittels dieser von einer nicht wirksamen Stellung in eine das nach dem Injektionsvorgang über das distale Ende 11 des Traggehäuses 2 vorragende Nadelende 21 abdeckende Stellung verlagerbar. Die Auslösung der zweiten Antriebsbaugruppe 8 sowie die Verstellung des Nadelschutzelements 7 wird ebenfalls nachfolgend noch detailliert beschrieben werden. Das Nadelschutzelement 7 mitsamt der zweiten Antriebsbaugruppe 8 ist im Bereich der Außenseite des Traggehäuses 2 angeordnet und zumindest bereichsweise an diesem abgestützt.

Das Traggehäuse 2 ist über den größten Teil seiner Längserstreckung zwischen dem distalen Ende 11 und dem proximalen Ende 12 von der zuvor beschriebenen Aktivierungshülse 9 zusätzlich umgeben bzw. in dieser aufgenommen. Dabei überragt lediglich das distale Ende 11 des Traggehäuses 2 die Aktivierungshülse 9 in axialer Richtung. Hier ist unter dem Begriff "über den größten Teil" seiner Längserstreckung zu verstehen, dass sich die Aktivierungshülse 9 bezüglich der Länge des Traggehäuses 2 über eine Längserstreckung in einer unteren Grenze von zumindest 50 % und einer oberen Grenze bis zu 100 % erstreckt. Dabei sind Werte der Längserstreckung von zumindest 50 %, bevorzugt von zumindest 60 %, insbesondere von zumindest 70 %, bevorzugt von zumindest 80 %, bevorzugt von zumindest 90 %, besonders bevorzugt von zumindest 95 % vorteilhaft. Da nahezu das gesamte Traggehäuse 2 von der Aktivierungshülse 9 aufgenommen bzw. innerhalb dieser angeordnet ist, ist das Nadelschutzelement 7 mit der mit dieser zusammenwirkenden zweiten Antriebsbaugruppe 8 im Axialschnitt gesehen, radial zwischen dem Traggehäuse 2 und der Aktivierungshülse 9 angeordnet. Dadurch kann auf die zweite Antriebsbaugruppe 8 keine Einflussnahme für deren Auslösung bzw. Deaktivierung genommen werden. Die Aktivierungshülse 9 kann auch als Auslösehülse bezeichnet werden. Weiters dient diese nicht nur für die Funktion der Aktivierung bzw. Auslösung der ersten Antriebsbaugruppe 4 sondern auch für den Benutzer die gesamte Injektionsvorrichtung 1 zu halten und auch während des gesamten Abgabevorganges unverändert weiter daran zu halten.

Das Traggehäuse 2 umfasst seinerseits einen vorderen und einen hinteren Traggehäuseteil 27, 28, wobei der vordere Traggehäuseteil 27 dem distalen Enden 11 und somit dem Patienten 10 zugewendet ist. Der hintere Traggehäuseteil 28 ist dem proximalen Ende 12 näher liegend angeordnet. Die beiden Traggehäuseteile 27, 28 umgrenzen bzw. definieren jeweils einen vorderen und hinteren Aufnahmeraum 29, 30 und sind in einem zwischen diesen angeordneten Verbindungsabschnitt 31 über eine Kupplungsvorrichtung 32 in Richtung der Längsachse 13 in zwei zueinander unterschiedlichen Längspositionen in einer ersten und einer zweiten Kupplungsstellung miteinander kuppelbar.

In der Fig. 1 ist die erste Kupplungsstellung dargestellt, wobei die beiden Traggehäuseteile 27, 28 bezüglich der zweiten Kupplungsstellung eine dazu größere Längserstreckung in Richtung der Längsachse 13 aufweisen. Weiters ist noch gezeigt, dass im Verbindungsabschnitt 31 und der dort angeordneten Kupplungsvorrichtung 32 der vordere Traggehäuseteil 27 den hinteren Traggehäuseteil 28 übergreift.

Die Kupplungsvorrichtung 32 weist an den beiden Traggehäuseteilen 27, 28 jeweils zusammenwirkende Arretierelemente 33, 34 auf, welche derart ausgebildet sind, dass die beiden Traggehäuseteile 27, 28 jeweils in den beiden Kupplungsstellungen in zueinander gegensinnigen Bewegungsrichtung arretiert sind. Unter gegensinniger Bewegungsrichtung wird hier eine Arretierung verstanden, die ein auseinander bewegen der beiden Traggehäuseteile 27, 28 in Richtung der Längsachse 13 verhindern, jedoch ein aufeinander zu bewegen von der ersten Kupplungsstellung in die zweite Kupplungsstellung ermöglichen.

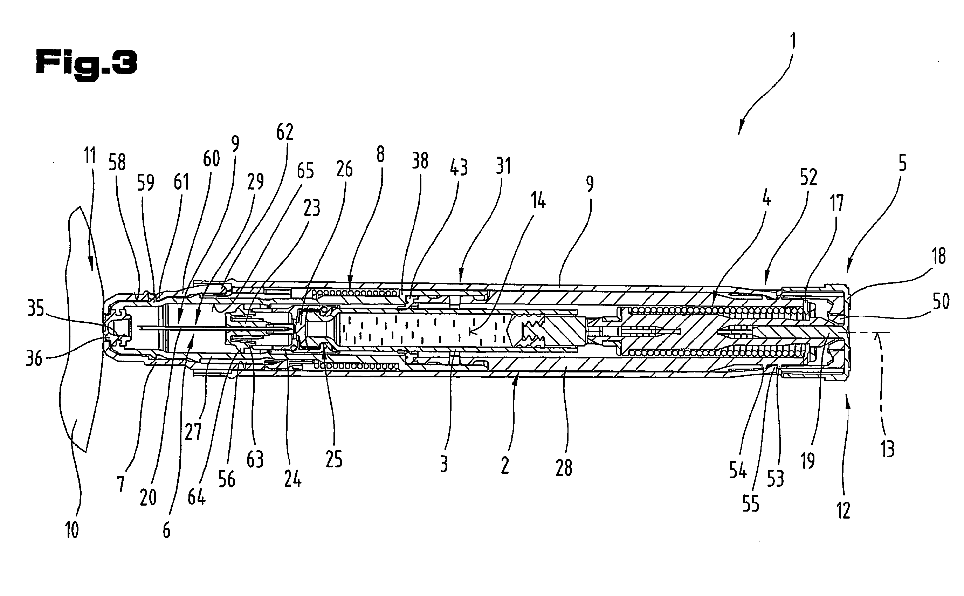

Die ersten Arretierelemente 33 der Kupplungsvorrichtung 32 sind beispielsweise im Bereich des vorderen Traggehäuseteils 27 durch Ausnehmungen bzw. Durchbrüche in der Wand des vorderen Traggehäuseteils 27 ausgebildet. Bevorzugt werden in Axialrichtung gesehen für die beiden Kupplungsstellungen mehrere hintereinander angeordnete Arretierelemente 33 vorgesehen. Das oder die weiteren Arretierelemente 34 der Kupplungsvorrichtung 32 des hinteren Traggehäuseteils 28 können durch in die Ausnehmungen bzw. Durchbrüche eingreifende Rastnasen bzw. Rastvorsprünge gebildet sein, wobei bevorzugt wiederum in Richtung der Längsachse 13 gesehen, mehrere dieser Arretierelemente 34 vorgesehen sind. In der ersten Kupplungsstellung, in welcher die beiden Traggehäuseteile 27, 28 eine größere Längserstreckung aufweisen als in der zweiten Kupplungsstellung, stehen jeweils nur die vorderen Arretierelemente 34 mit den hinteren Arretierelementen 33 des vorderen Traggehäuseteils 27 in Eingriff. Die zweite Kupplungsstellung ist in der Fig. 3 in einer zur Fig. 1 gedrehten anderen Schnittebene dargestellt. Dabei kommen in der zweiten Kupplungsstellung jeweils die beiden hintereinander angeordneten Arretierelemente 34 des hinteren Traggehäuseteils 28 in die korrespondierenden Arretierelemente 33 des vorderen Traggehäuseteils 37 in Eingriff. Somit wird in beiden Stellungen ein voneinander Entfernen der beiden Traggehäuseteile 27, 28 verhindert.

Im Bereich des distalen Endes 11 weist das Traggehäuse 2 bzw. der vordere Traggehäuseteil 27 eine den Durchtritt des Nadelendes 21 der Nadelanordnung 6 ermöglichende Öffnung 35 auf. In dieser Öffnung 35 ist ein durchstechbarer Dichtstopfen 36 in einer den vorderen Aufnahmeraum 29 gegenüber der äußeren Umgebung dichtenden Stellung angeordnet.

Der vordere Traggehäuseteil 27 weist im Verbindungsabschnitt 31 mit dem hinteren Traggehäuseteil 28 zumindest eine Durchsetzung 37 auf, welche einen Zugang von der Außenseite des Traggehäuse 2 zumindest in den vorderen Aufnahmeraum 29 mit der darin angeordneten Nadelanordnung 6 ermöglicht. Die Durchsetzung 37 ist dabei in dem den hinteren Traggehäuseteil 28 übergreifenden Wandabschnitt des vorderen Traggehäuseteils 27 angeordnet bzw. vorgesehen. Unmittelbar benachbart zu der bzw. den Durchsetzungen 37 endet der den hinteren Traggehäuseteil 28 übergreifende Verbindungsabschnitt 31 mit einem hin auf die Längsachse 13 rückspringenden Wandteil 38, welcher bevorzugt in einer senkrecht zur Längsachse 13 ausgerichteten Ebene verlaufend angeordnet ist. Dieser Wandteil 38 weist eine dem hinteren Traggehäuseteil 28 zugewendete Stirnwand 39 auf, welche ebenfalls bevorzugt in der senkrecht zur Längsachse 13 ausgerichteten Ebene verlaufend angeordnet ist. An dem Wandteil 38 ist weiters an der vom hinteren Traggehäuseteil 28 abgewendeten Seite die zweite Antriebsbaugruppe 8 mit einem weiteren Antriebsmittel 40 daran abgestützt. Das Antriebsmittel 40 kann wiederum durch eine vorgespannte Druckfeder oder ein ähnliches Federelement gebildet sein.

Weiters kann an der rückspringenden Stirnwand 39 des Wandteils 38 ein im Axialschnitt gesehen keilförmig ausgebildeter und über den Umfang durchlaufend ausgebildeter Ansatz 41 angeordnet sein. Die keilförmige Verjüngung ist dabei hin in Richtung auf den hinteren Traggehäuseteil 28 gerichtet.

Am hinteren Traggehäuseteil 28 ist an seinem dem vorderen Traggehäuseteil 27 zugewendeten Ende 42 ein über den Umfang durchlaufend ausgebildetes Dichtelement 43 angeordnet bzw. daran gehaltert. Das Dichtelement 43 ist bevorzugt an einer Stirnseite des hinteren Traggehäuseteils 28 angeordnet und daran gehaltert. Durch das umlaufend ausgebildete Dichtelement 43 liegt dieses sowohl an einer Außenfläche 44 der Karpule 3 als auch an einer Innenfläche 45 des vorderen Traggehäuseteils 27, insbesondere im Verbindungsabschnitt 31 an. Dieses Anliegen erfolgt bevorzugt dichtend über den gesamten Umfang an beiden Flächen 44, 45, wobei in der ersten Kupplungsstellung der beiden Traggehäuseteile 27, 28, das Dichtelement 43 noch distanziert von der Stirnwand 39 des Wandteils 38 bzw. dem an der Stirnwand 39 ausgebildeten Ansatz 41 angeordnet ist.

Der vordere Traggehäuseteil 27 weist einen in etwa zylinderförmig ausgebildeten Mantel 46 auf, der im Bereich des distalen Endes 11 die zuvor beschriebene Öffnung 35 aufweist, welche mit dem Dichtstopfen 36 verschlossen ist. Der Mantel 46 ist durchlaufend und somit dicht bis hin zum Verbindungsabschnitt 31 ausgebildet. In diesem sind lediglich die Durchsetzungen 37 vorgesehen, durch welche in der ersten Kupplungsstellung der beiden Traggehäuseteile 27, 28 eine Strömungsverbindung in den vorderen Aufnahmeraum 29 des vorderen Traggehäuseteils 27 ausgebildet wird. Es kann hier über den Umfang gesehen nur eine oder aber auch mehrere der Durchsetzungen 37 vorgesehen sein, welche ausgehend von der Außenseite des vorderen Traggehäuseteils 27 in den vorderen Aufnahmeraum 29 einmünden. So wird in der ersten Kupplungsstellung der vordere Aufnahmeraum 29 des vorderen Traggehäuseteils 27 einerseits durch den Mantel 46, den in der Öffnung 35 angeordneten Dichtstopfen 36, das Dichtelement 43 sowie die in den vorderen Aufnahmeraum 29 hineinragende Karpule 3 dichtend umgrenzt und lediglich durch die Durchsetzung 37 eine Strömungsverbindung ermöglicht.

Unabhängig davon wäre es aber auch noch möglich, nicht nur den vorderen Aufnahmeraum 29 über die Durchsetzungen 37 für die Sterilisation zugänglich zu machen, sondern zusätzlich auch noch einen Zugang zum hinteren Aufnahmeraum 30 zu schaffen. Dies könnte dadurch realisiert werden, dass das Dichtelement 43 über den Umfang gesehen nicht vollständig an der Außenfläche 44 der Karpule 3 anliegt. Die Abstützung der Karpule 3 am hinteren Traggehäuseteil 28 kann über einzelne über den Umfang verteilte Stützstege erfolgen, welche hier nicht näher dargestellt worden sind. Weiters kann durch die geteilte Ausbildung des Traggehäuses 2 in den vorderen sowie hinteren Traggehäuseteil 27, 28 eine einfache Sterilisation mittels Begasung durchgeführt werden und anschließend durch einen entsprechenden Abdichtungsvorgang des sterilen Innenraums dieser über einen längeren Zeitraum auch aufrecht erhalten werden.

In der zweiten Kupplungsstellung der beiden Traggehäuseteile 27, 28 liegt das Dichtelement 43 dichtend an der Stirnwand 39 des Wandteils 38 bzw. dem daran angeordneten Ansatz 41 an. Zur Erzielung einer besseren umlaufenden Dichtung ist der zuvor beschriebene keilförmige Ansatz 41 vorgesehen, welcher sich in axialer Richtung in das Dichtelement 43 während dessen Anlage daran hineindrückt.

Durch die gegenseitige Verlagerung der beiden Traggehäuseteile 27, 28 aufeinander zu, wird das Dichtelement 43 an der Durchsetzung 37 vorbei geschoben und es liegt ein vollständig abgedichteter vorderer Aufnahmeraum 29 vor. Darüber hinaus kann das umlaufende Dichtelement 43 auch als dämpfende Halterung für die im Traggehäuse 2 aufgenommene Karpule 3 mit dem Medikament 14 dienen. Dadurch können Stöße und Schlagbeanspruchungen, ausgehend von der äußeren Aktivierungshülse 9 hin zur Karpule 3 mit dem Medikament 14 gedämpft bzw. vollständig reduziert bzw. abgefangen werden.

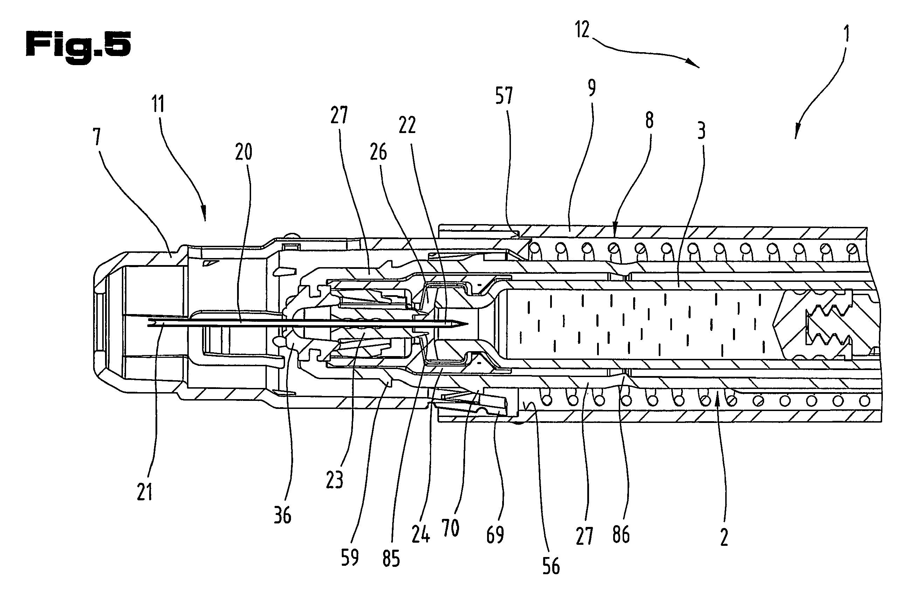

Eine weitere Dämpfungsvorrichtung 86 ist aus einer Zusammenschau der Fig. 1 und 5 zu ersehen. Diese ist an der Innenfläche 45 des Traggehäuses 2, insbesondere dem vorderen Traggehäuseteil 27, als in den lichten Querschnitt hineinragender Flansch oder als Einschnürung des Mantels 46 ausgebildet. Die Anordnung erfolgt in Axialrichtung gesehen in der Aufbewahrungsstellung im Bereich bzw. Abschnitt des Nadelträgers 23 bzw. des Führungselements 24. Bei einem Falltest könnte sich so die Karpule 3 bzw. der Nadelträger 23 und/oder das Führungselement 24 daran abstützen und so eine unbeabsichtigte Weiterbewegung der Karpule 3 hin in Richtung auf das distale Ende 11 gestoppt werden. Die Dämpfungsvorrichtung 86 bildet somit einen mechanischen Widerstand gegen eine axiale Bewegung aus.

Der Zugang über die Durchsetzung 37 in der ersten Kupplungsstellung dient zur Sterilisierung des vorderen Aufnahmeraums 29 mitsamt der darin angeordneten Nadelanordnung 6. Da während der Sterilisation bereits die Karpule 3 mit dem darin bevorrateten Medikament 14 innerhalb der Injektionsvorrichtung 1 angeordnet ist, kommen nur wenige Sterilisationsmöglichkeiten in Frage. Eine Sterilisation mit Hitzeeinwirkung oder einer Bestrahlung mit Elektronenstrahlen sollte aus diesen Gründen vermieden werden, um das Medikament 14 nicht negativ zu beeinflussen. Im vorliegenden Ausführungsbeispiel wird bevorzugt eine Begasung mittels Ethylenoxid über die in dem Verbindungsabschnitt 31 angeordnete Durchsetzung 37 gewählt. Diese Begasung kann auch als ETO-Begasung bezeichnet werden und ist hinlänglich bekannt. Nach dem Sterilisationsvorgang werden die beiden Traggehäuseteile 27, 28 von deren ersten Kupplungsstellung in die zweite Kupplungsstellung gegeneinander verstellt, wodurch, wie bereits zuvor beschrieben, das umlaufende Dichtelement 43 den vollständigen Abschluss des vorderen Aufnahmeraums 29 gegenüber den äußeren Umgebungsbedingungen sicherstellt. Dadurch wird die gesamte Nadelanordnung 6 sowie der Karpulenverschluss 26 an der dem vorderen Aufnahmeraum 29 zugewendeten Seite sterilisiert und liegt bis zum Gebrauch bzw. dem Einsatz der Injektionsvorrichtung 1 in diesem Zustand vor.

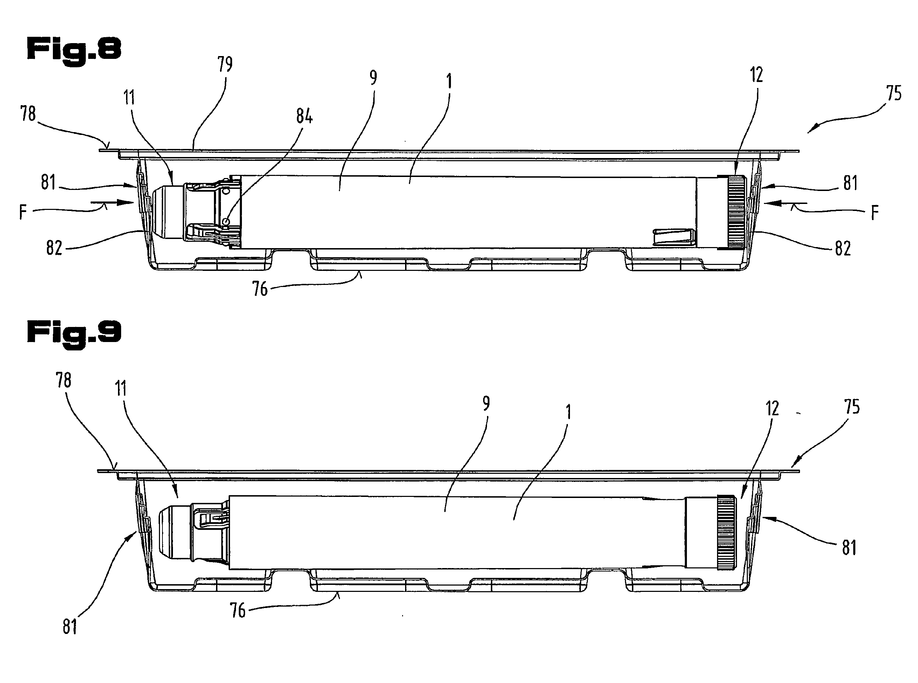

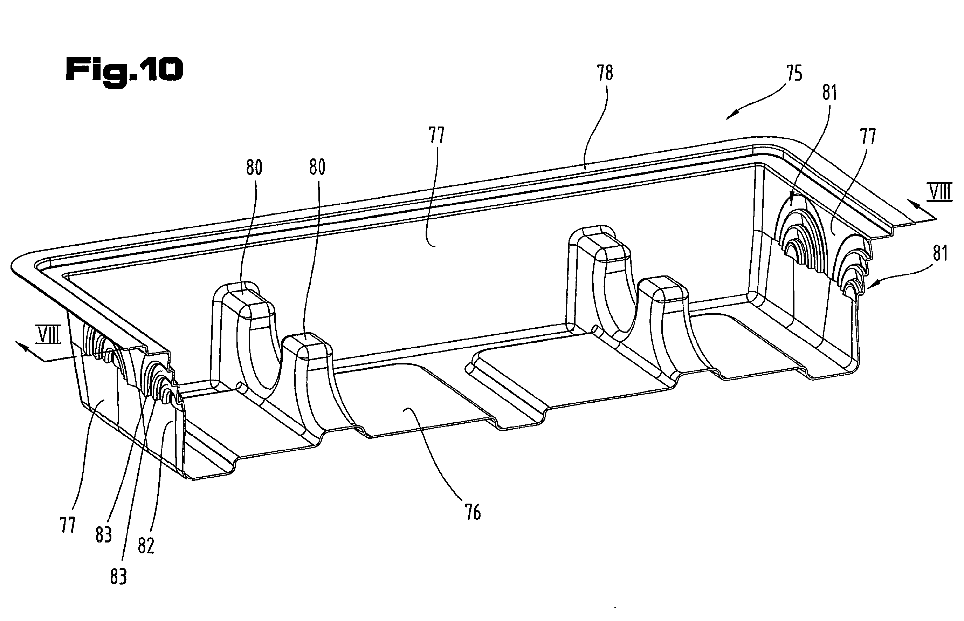

Weiters ist es möglich, dass vor dem Durchführen der Sterilisation mittels Begasung die Injektionsvorrichtung 1 von einer nicht näher dargestellten Umhüllung vollständig umschlossen wird. Dabei kann beispielsweise die Umhüllung aus einem papierfließartigen Faserfunktionstextil aus thermisch verschweißten Fasern aus Polyethylen mit hoher Dichte (HDPE) gewählt werden. Das papierfließartige Faserfunktionstextil wird beispielsweise von der Firma DuPont unter dem Markennamen TYVEK vertrieben und weist die Eigenschaft auf, dass einerseits der Durchtritt des für die Begasung notwendigen Ethylenoxids ermöglicht wird, jedoch der von Keimen bzw. Bakterien gesichert verhindert ist. So kann beispielsweise die in der ersten Kupplungsstellung des vorderen und hinteren Traggehäuseteils 27, 28 befindliche Injektionsvorrichtung 1 in einfacher und oder aber auch mehrfacher Anordnung von der Umhüllung umgeben werden. Anschließend daran wird an einem dafür geeigneten Ort die Sterilisation mittels der Begasung durchgeführt. Nachfolgend an die Sterilisation werden in einem eigenen Arbeitsgang die beiden Traggehäuseteile 27, 28 in die zweite Kupplungsstellung verstellt und dadurch der vordere Aufnahmeraum 29 vollständig dichtend gegenüber den äußeren Umgebungsbedingungen abgeschossen, wie dies zuvor bereits detailliert beschrieben worden ist. Werden mehrere Injektionsvorrichtung 1 in einem Tray bzw. einer nachfolgend noch näher beschriebenen Aufnahmeschale aufbewahrt und mittels einer Abdeckung an der Aufnahmeschale abgedeckt bzw. von der Umhüllung umgeben, kann das Tray jeweils an den den beiden Enden 11, 12 zugewendeten Wandteilen leicht verformbare Wandabschnitte aufweisen, welche ein Zusammenschieben der beiden Traggehäuseteile 27, 28 gegeneinander von der ersten Kupplungsstellung in die zweite Kupplungsstellung ermöglichen, ohne dass das Tray dabei beschädigt wird. Dies ist in den Fig. 8 bis 10 näher beschrieben.
Die Aktivierungshülse 9 ist an ihrem proximalen Ende 12 derart ausgebildet, dass diese zur Aufnahme bzw. der Kupplung der Sicherungskappe 18 dient. Dazu weist eine Hülsenwand 47 der überwiegend zylinder- bzw. rohrförmig ausgebildeten Aktivierungshülse 9 im Bereich ihres proximalen Endes 12 eine reduzierte Wandstärke auf und bildet somit einen Aufnahmebereich an der Außenseite der Hülsenwand 47 aus. Ein Kappenmantel 49 übergreift an der von der Längsachse 13 abgewendeten Seite den Aufnahmebereich 48 und ist dabei bevorzugt in etwa ebenflächig zur Außenseite der Hülsenwand 47 verlaufend ausgebildet.

Weiters weist die Aktivierungshülse 9 an ihrem proximalen Ende 12 im Bereich der Längsachse 13 zumindest ein keilförmig ausgebildetes Aktivierungselement 50 auf. Dieses Aktivierungselement 50 kann über den Umfang durchlaufend ausgebildet sein und an der der Antriebsbaugruppe 4 zugewendeten Seite eine sich kegelig auf die von der Antriebsbaugruppe 4 abgewendete Seite verjüngende Kegelfläche 51 aufweisen. Nach Entfernung der Sicherungskappe 18 mit ihrem Sicherungsstift 19 ist es dann bei einer relativen Verlagerung der Aktivierungshülse 9 bezüglich dem Traggehäuse 2 möglich, die gegengleich zur Kegelfläche 51 ausgebildeten Enden der Haltearme 16 radial nach innen - also in Richtung auf die Längsachse 13 hin - zu verlagern und dadurch die Freigabe von der Haltescheibe 17 zu erzielen. Dieser Auslösevorgang ist bei derartigen Injektionsvorrichtungen 1 üblich und zählt somit zum allgemeinen Fachwissen. Deshalb wird hier nicht mehr auf die detaillierte Ausbildung dieses Aktivierungs- bzw. Auslösemechanismuses für die erste Antriebsbaugruppe 4 eingegangen.

Wie bereits zuvor beschrieben, ist in der Fig. 3 die sterilisierte Aufbewahrungsstellung der Injektionsvorrichtung 1 gezeigt, bei der der vordere Aufnahmeraum 29 sowie die später mit dem Patienten 10 in Verbindung bzw. Kontakt kommende Kanüle 20 für den Einstich und die damit verbundene Abgabe des Medikamentes 14 im völlig sterilisierten Zustand vorliegt. Weiters ist im Bereich des proximalen Endes 12 der Injektionsvorrichtung 1 vereinfacht eine Rastvorrichtung 52 dargestellt, welche eine Rastwirkung zwischen dem Traggehäuse 2, insbesondere dem hinteren Traggehäuseteil 28 und der Aktivierungshülse 9, bewirkt.

So weist der hintere Traggehäuseteil 28 an seiner äußeren Oberfläche und im Bereich des proximalen Endes 12 zumindest zwei in Richtung der Längsachse 13 voneinander distanzierte Rastnasen 53, 54 auf, welche mit einem an der Aktivierungshülse 9 angeordneten Rastelement 55 in zwei unterschiedlichen Längspositionierungen zusammenwirken. Das an der Aktivierungshülse 9 ausgebildete Rastelement 55 ist bevorzugt ein Bestandteil der Hülsenwand 47 und kann beispielsweise mit einem federn ausgebildeten Rastarm in Verbindung stehen bzw. durch diesen gebildet sein. Die erste Rastnase 53 ist dabei dem proximalen Ende 12 näher liegend angeordnet als die weitere Rastnase 54. Die Distanzierung der beiden Rastnasen 53, 54 entspricht dabei bevorzugt jenem Auslöseweg der Aktivierungshülse 9 relativ gegenüber dem Traggehäuse 2 der notwendig ist, um zumindest die erste Antriebsbaugruppe 4 freizugeben und damit für den Injektionsvorgang zu aktivieren. Die beiden Rastnasen 53, 54 definieren die relativen Stellungen des Traggehäuses 2 bezüglich der Aktivierungshülse 9 einerseits in der Aufbewahrungsstellung und andererseits in der Injektionsstellung. Dabei ist ausschließlich eine relative Längsverstellung der Aktivierungshülse 9 bezüglich des Traggehäuses 2 von der Aufbewahrungsstellung hin zur Injektionsstellung möglich, jedoch eine in dazu entgegen gesetzter Richtung relative Längsverstellung gesichert verhindert.

Die Aktivierung bzw. Auslösung der Injektionsvorrichtung 1 für den Injektionsvorgang von der Aufbewahrungsstellung hin zur Injektionsstellung erfolgt dadurch, dass die gesamte Injektionsvorrichtung 1 von der Hand eines Benutzers an der Aktivierungshülse 9 gehalten wird und dann die Sicherungskappe 18 vom proximalen Ende 12 der Aktivierungshülse 9 abgezogen wird. Damit ist die Injektionsvorrichtung 1 für die Auslösung und den nachfolgenden Injektionsvorgang aktiviert. Anschließend daran ist die gesamte Injektionsvorrichtung 1 mit ihrem distalen Ende 11 an jener Stelle des Patienten 10 zu positionieren, an welcher das Medikament 14 verabreicht werden soll. Dadurch stützt sich das distale Ende des Nadelschutzelements 7 sowie gegebenenfalls der am vorderen Traggehäuseteil 27 in der Öffnung 35 angeordnete Dichtstopfen 36 daran ab. Durch die Verlagerung der Aktivierungshülse 9 in Richtung des distalen Endes 11 und somit auf den Patienten 10 zu, erfolgt durch die Abstützung des Traggehäuses 2 am Körperteil des Patienten 10 eine relative Verlagerung der Aktivierungshülse 9 bezüglich dem Traggehäuse 2. Dadurch wird, wie bereits zuvor beschrieben, die erste Antriebsbaugruppe 4, insbesondere die verrastet gehaltenen Haltearme 16, durch das Aktivierungselement 50 radial nach innen verlagert und dadurch die Verrastung mit der Haltescheibe 17 gelöst.

Die relative axiale Verstellbewegung bewirkt, dass das federnde Rastelement 55 vom Eingriff mit der ersten Rastnase 53 außer Eingriff kommt, über die zweite Rastnase 54 hinweg gleitet und sich anschließend an dieser zweiten Rastnase 54 wiederum abstützt. Dadurch wird das Traggehäuse 2 relativ gegenüber der Aktivierungshülse 9 ortsfest positioniert gehaltert. Diese Arretierung erfolgt in beiden axialen Richtungen, wobei die erste Abstützung zwischen den Haltearmen 16 und dem Aktivierungselement 50 und die zweite Abstützung zwischen der zweiten Rastnase 54 und dem Rastelement 55 erfolgt.

Weiters weist die Aktivierungshülse 9 im Bereich des distalen Endes 11 sowie an deren Innenfläche 56 eine Anschlagfläche 57 auf, welche bevorzugt in senkrechter Richtung bezüglich der Längsachse 13 ausgerichtet ist. Diese Anschlagfläche 57 kann bevorzugt durchlaufend über den inneren Umfang der Aktivierungshülse 9 ausgebildet bzw. angeordnet sein und wird bevorzugt durch eine zylinderförmige bzw. rohrförmige Ausnehmung in der Hülsenwand 47 ausgebildet.

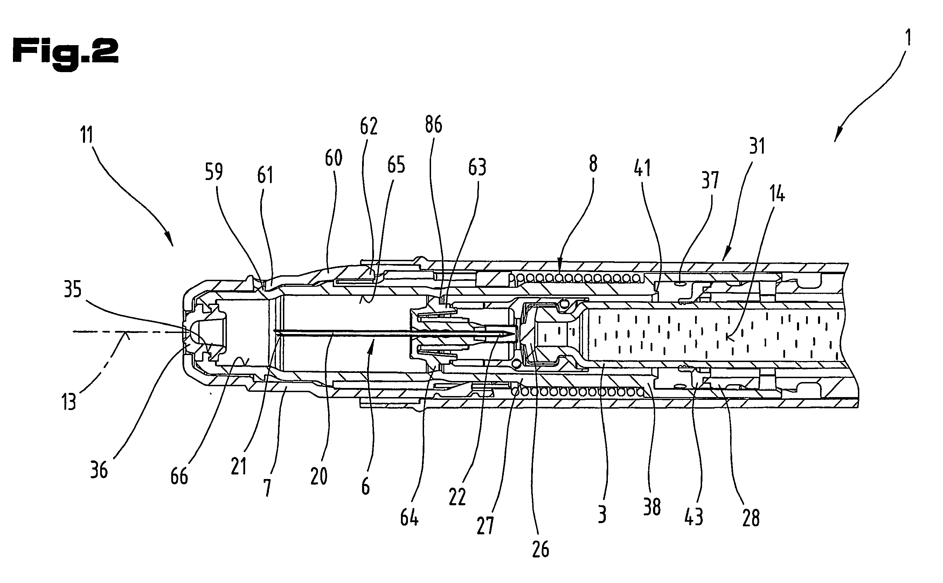

Auch das Nadelschutzelement 7 ist unter Vorspannung der zweiten Antriebsbaugruppe 8 gegenüber dem Traggehäuse 2, insbesondere dem vorderen Traggehäuseteil 27 in der Aufbewahrungsstellung arretiert festgelegt. Diese relative und lösbare Fixierung ist am Besten aus den Fig. 2 und 3 im Bereich des distalen Endes 11 zu entnehmen. Das Traggehäuse 2 bzw. der vordere Traggehäuseteil 27 weist an seinem distalen Ende 11 an seiner äußeren Oberfläche 58 ein bevorzugt rundum durchlaufendes Anschlagelement 59 auf. Am Nadelschutzelement 7 ist korrespondierend dazu zumindest ein wippenartig ausgebildetes Hebelelement 60 vorgesehen, welches in Richtung der Längsachse 13 voneinander distanzierte Hebelenden 61, 62 aufweist. Das dem distalen Ende 11 näher liegende Hebelende 61 stützt sich in der Aufbewahrungsstellung der Injektionsvorrichtung 1 an dem zuvor beschriebenen Anschlagelement 59 des vorderen Traggehäuseteils 27 des Traggehäuses 2 ab. Das weitere Hebelende 62 ragt in die zuvor beschriebene Ausnehmung der Aktivierungshülse 9 hinein sowie in Richtung auf die Anschlagfläche 57 zu. Je nach dem gewählten Abstand zwischen dem Hebelende 63 und der Anschlagfläche 57 kann der später noch beschriebene Auslösezeitpunkt für das Nadelschutzelement 7 bzw. der mit dieser zusammenwirkenden zweiten Antriebsbaugruppe 8 variiert werden. Die zweite Antriebsbaugruppe 8 mit deren federartig ausgebildeten Antriebsmittel 40 ist im Bereich seines dem proximalen Ende 12 zugewendeten Endbereiches am rückspringenden Wandteil 38 abgestützt. Weiters stützt sich die zweite Antriebsbaugruppe 8, insbesondere das Antriebsmittel 40, mit seinem dem distalen Ende 11 zugewendeten Endbereich an einer dem proximalen Ende 12 zugewendeten Stirnwand des Nadelschutzelements 7 ab. Damit ist das Antriebsmittel 40 der zweiten Antriebsbaugruppe 8 einerseits in radialer Richtung gesehen zwischen dem Traggehäuse 2 und der Aktivierungshülse 9 und andererseits in axialer Richtung gesehen am rückspringenden Wandteil 38 des Traggehäuses 2 sowie der Stirnwand des Nadelschutzelements 7 angeordnet. Durch die Abstützung an der Stirnwand des Nadelschutzelements 7 ist das Antriebsmittel 40 der zweiten Antriebsbaugruppe 8 in axialer Richtung gesehen hinterhalb des Nadelschutzelements 7 und somit auf der dem proximalen Ende 12 zugewendeten Seite bzw. dem dort ausgebildeten Endabschnitt angeordnet. Dadurch erzeugt es auf das Nadelschutzelement 7 eine in Richtung auf das distale Ende 11 gerichtete Druckkraft. Diese Druckkraft wird vom Nadelschutzelement 7 über das daran angeordnete bzw. ausgebildete Hebelelement 60 auf das am Traggehäuse 2 angeordnete Anschlagelement 59 übertragen und ist somit dort abgestützt.

Wie bereits zuvor beschrieben, ist die gesamte Nadelanordnung 6, insbesondere die Kanüle 20 mit ihrem dem Karpulenverschluss 26 zugewendeten Nadelende 22, distanziert von diesem in der Aufbewahrungsstellung angeordnet. Weiters ist der Nadelträger 23 im Führungselement 24 axial verschiebbar gehalten bzw. darin gelagert, wobei in der Aufbewahrungsstellung der Nadelträger 23 über zumindest ein lösbares Halteelement 63 relativ bezüglich dem Führungselement 24 in seiner Stellung daran festgelegt ist. Dies ist am Besten aus der Darstellung der Fig. 1 zu ersehen.

Wie nun besser aus den Fig. 2 und 3 zu ersehen ist, ist dem Halteelement 63 des Nadelträgers 23 eine Stellnocke 64 zugeordnet bzw. daran angeordnet, welche den Nadelträger 23 an die von der Längsachse 13 abgewendete Seite überragt. Der vordere Traggehäuseteil 27 des Traggehäuses 2 weist in diesem Abschnitt eine innere lichte Abmessung bzw. einen Querschnitt auf, welcher in etwa zylindrisch ausgebildet ist und dabei die Stellnocke 64 unmittelbar benachbart bis leicht anliegend an einer Innenwand 65 des hülsenförmig ausgebildeten Traggehäuseteils 27 angeordnet. Wird nun mittels der ersten Antriebsbaugruppe 4 die Karpule 3 mitsamt der Nadelanordnung 6 hin in Richtung auf das distale Ende 11 zu bewegt, durchsticht zuerst das dem distalen Ende 11 zugewendete Nadelende 21 den in der Öffnung 35 des vorderen Traggehäuseteils 27 angeordneten Dichtstopfen 36 und tritt dann unmittelbar in den zur Abgabe vorgesehenen Oberflächenabschnitt des Patienten 10 ein.

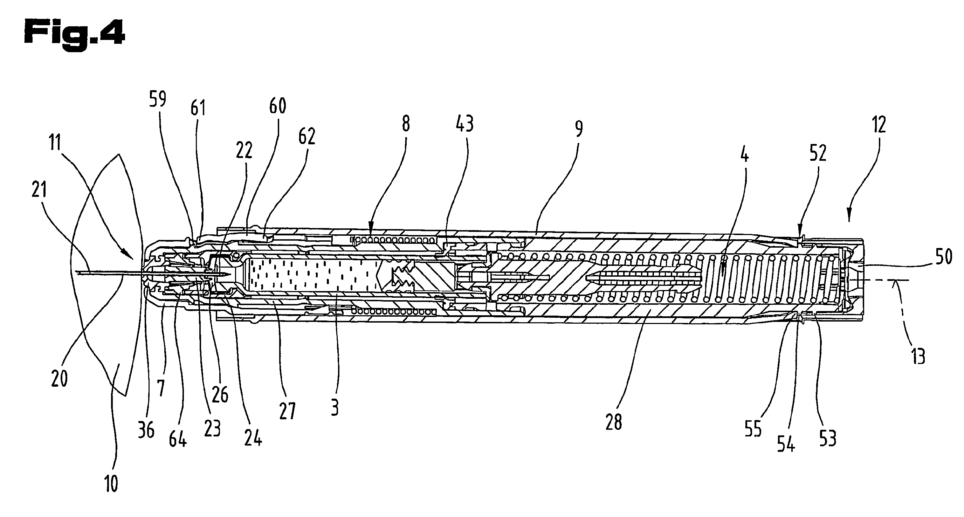

Die aktivierte und eingestochene Stellung der Kanüle 20 in den Patienten 10 ist am Besten aus der Fig. 4 zu sehen. Dabei wurde die Karpule 3 mitsamt der Nadelanordnung 6 hin in Richtung auf das distale Ende 11 verlagert. Weiters ist im Bereich des distalen Endes 11 an der Innenwand 65 des vorderen Traggehäuseteils 27 eine in Richtung auf die Längsachse 13 vorragende Steuerkurve 66 ausgebildet, welche beispielsweise durch eine Verengung des den vorderen Traggehäuseteils 27 ausbildenden Wandteils gebildet ist. Ebenso wäre aber auch die Ausbildung einer Rippe bzw. eines Steges an der Innenwand 65 denkbar. Die in Richtung auf die Längsachse 13 hin versetzte Steuerkurve 66 mit der damit verbundenen Verkleinerung bzw. Verengung des Querschnittes kommt im Zuge des Verschiebeweges der Nadelanordnung 6 mit der Stellnocke 64 in Eingriff und wird mitsamt dem Halteelement 63 radial nach innen in Richtung auf die Längsachse 13 hin verlagert. Durch diese Verlagerung kommt das Halteelement 63 außer Eingriff mit dem Führungselement 24. Bedingt durch diese Freigabe und der mechanischen Anlage des Nadelträgers 23 an dem im distalen Ende 11 angeordneten Dichtstopfen 36, kommt es zu einer weiteren relativen Verlagerung zwischen dem Nadelträger 23 und dem Führungselement 24. Durch diese relative Verlagerung durchsticht das dem proximalen Ende 12 zugewendete Nadelende 22 den Karpulenverschluss 26, wodurch nun eine Strömungsverbindung zwischen dem Innenraum der Karpule 3 über der Kanüle 20 hin zur Einstichstelle des Patienten 10 geschaffen wird. Durch den mechanischen Anschlag des Nadelträgers 23 am Dichtstopfen 36 und dem Beenden der relativen Verstellung zwischen dem Nadelträger 23 und dem Führungselement 24, wird auch die Karpule 3 ortsfest bezüglich des Traggehäuses 2 festgelegt. Der Dichtstopfen 36 ist dabei an dem dem Nadelträger 23 bzw. dem Führungselement 24 zugewendeten Ende derart ausgebildet, dass dieser auch noch eine Dämpfungsfunktion für das Beenden der Verstellbewegung übernimmt und die zumeist aus Glas hergestellte Karpule 3 nicht durch die ruckartige Verstellbewegung und den damit verbundenen Aufprall am distalen Ende 11 zu Bruch geht. Deshalb ragt ein bevorzugt umlaufender Bund von der Öffnung 35 in Richtung des vorderen Aufnahmeraums 29 vor, welcher dabei mit der Nadelanordnung 6 in Kontakt kommt.

Durch die weitere Druckwirkung bzw. Kraftbeaufschlagung der Antriebsbaugruppe 4 hin in Richtung auf das distale Ende 11 wird ein an einer Kolbenstange 67 angeordneter Karpulenstopfen 68 in den Innenraum der Karpule 3 hinein gedrückt und damit das Medikament 14 über die zu diesem Zeitpunkt eingestochene Kanüle 20 in den Patienten 10 injiziert.

Im Zuge der relativen Verlagerung der Aktivierungshülse 9 bezüglich des Traggehäuses 2 und der damit verbundenen Auslösung der ersten Antriebsbaugruppe 4 kommt das dem proximalen Ende 12 zugewendete zweite Hebelende 62 des Hebelelements 60 mit der an der Innenfläche 56 der Aktivierungshülse 9 ausgebildeten Anschlagfläche 57 in Kontakt. Dadurch wird nun das dem proximalen Ende 12 zugewendete Hebelende 62 radial in Richtung auf die Längsachse 13 hin verschwenkt. In weiterer Folge bewirkt die wippenartige Lagerung des Hebelelements 60, dass das dem distalen Ende 11 zugewendete erste Hebelende 61 des Hebelelements 60 außer Eingriff mit dem Anschlagelement 59 durch eine radiale Verschwenkung auf die von der Längsachse 13 abgewendete Seite kommt. Damit wird die Abstützung des Nadelschutzelements 7 am Traggehäuse 2, insbesondere dem vorderen Traggehäuseteil 27, gelöst und gleichzeitig damit die zweite Antriebsbaugruppe 8 aktiviert. Dadurch wird nach dem Beenden des Injektionsvorganges und dem Entfernen der Injektionsvorrichtung 1 vom Patienten 10 sofort das Nadelschutzelement 7 mit der damit zusammenwirkenden Antriebsbaugruppe 8 von seiner nicht wirksamen Stellung in die das nach dem Injektionsvorgang über das distale Ende des Traggehäuses 2 vorragende Nadelende abdeckend Stellung verlagert. Vorteilhaft ist es, wenn mit der axialen Verstellung der Aktivierungshülse 9 relativ bezüglich des Traggehäuses 2 die gleichzeitige Freigabe sowohl der ersten als auch der zweiten Antriebsbaugruppe 4, 8 erfolgt.

In der Fig. 5 ist nun die geschützte Stellung der Kanüle 20 nach dem Injektionsvorgang und dem Abziehen von der Injektionsstelle des Patienten 10 innerhalb des Nadelschutzelements 7 gezeigt.

Um eine unbeabsichtigte Rückstellung des Nadelschutzelements 7 ausgehend von der schützenden und abdeckenden Stellung hin zu einer erneuten Freigabestellung des Nadelendes 21 zu verhindern, weist das Nadelschutzelement 7 weiters an seinem proximalen Ende 12 zumindest ein elastisch verformbar ausgebildetes Sicherungselement 69 auf, welches als elastisch verformbarer Hebel ausgebildet sein kann. Das Nadelschutzelement 7 wird während seiner relativen Verlagerung bezüglich des Traggehäuses 2 in die die Nadelanordnung 6 abdeckende Stellung durch die Antriebsbaugruppe 8 verlagert. Die Rückstellung des Nadelschutzelements 7 hin in Richtung auf das proximale Ende 12 wird durch die Abstützung des Sicherungselements 69 an der an der Innenfläche 56 der Aktivierungshülse 9 ausgebildeten Anschlagfläche 57 verhindert. Dabei wird das Nadelschutzelement 7 mittels der zweiten Antriebsbaugruppe 8 über einen vorbestimmten Verstellweg bis zu einem mechanischen Anschlag hin verstellt. Das Sicherungselement 69 ragt dabei in den Freiraum vor der Anschlagfläche 57 hinein. Weiters kann das Sicherungselement 69 in einem gewissen Abstand vor der Anschlagfläche 57 bzw. aber auch unmittelbar daran anliegend angeordnet sein. Die Verraststellung des Sicherungselements 69 wird bei der die Nadelanordnung 6 abdeckenden Stellung des Nadelschutzelements 7 zusätzlich durch ein am Traggehäuse 2 angeordnetes Stellelement 70 unterstützt bzw. gesichert. Das Stellelement 70 kann als umlaufender stegförmiger Ansatz am Traggehäuse 2 bzw. dem vorderen Traggehäuseteil 27 ausgebildet sein, wobei das Stellelement 70 während der relativen Verstellung des Nadelschutzelements 7 bezüglich des Traggehäuses 2 das Sicherungselement 69 radial auf die von der Längsachse 13 abgewendete Seite drückt bzw. verstellt. Durch diese mechanische Verrastung des Sicherungselements 69 an der Anschlagfläche 57 sowie der radialen Vorspannung durch das Stellelement 70 wird eine gesicherte Abdeckung der Kanüle 20 durch das Nadelschutzelement 7 gewährleistet.

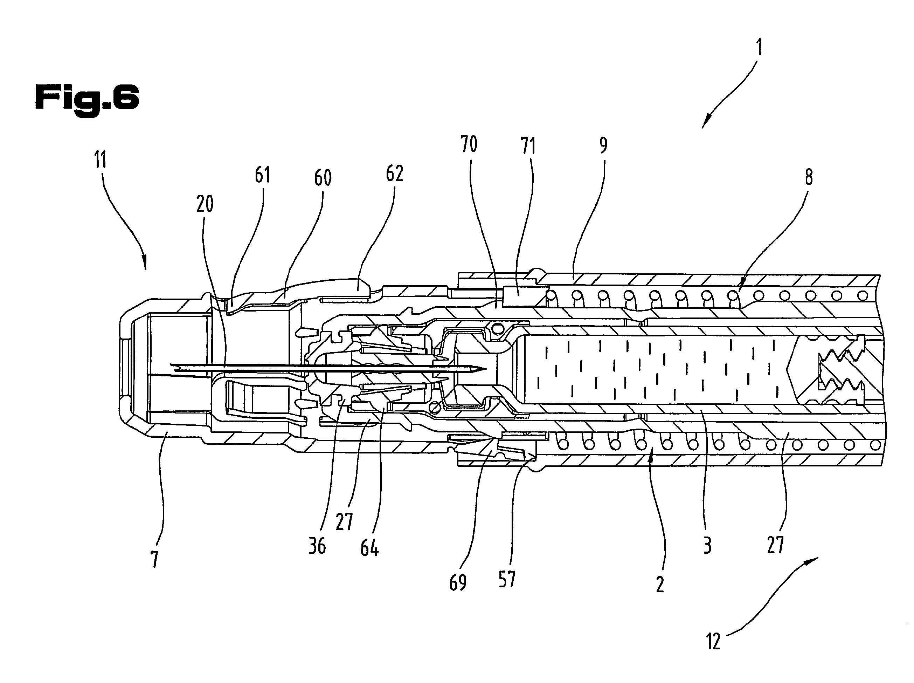

Die axiale Verstellbewegung des Nadelschutzelements 7 relativ gegenüber dem Traggehäuse 2 kann durch einen formschlüssigen Anschlag zwischen einem am proximalen Ende 12 angeordneten Rastteil 71 und dem am Traggehäuse 2 angeordneten Stellelement 70 in Richtung auf das distale Ende 11 beendet werden. Dadurch wird das Nadelschutzelement 7 über einen vorbestimmten Verstellweg hin in Richtung auf das distale Ende 11 verstellt, wobei in dieser Stellung die Kanüle 20 der Nadelanordnung 6 vollständig abdeckt ist. Dies ist am Besten aus der Fig. 6 zu entnehmen.

Weiters ist aus der Fig. 5 noch zu entnehmen, dass der Nadelträger 23 an seiner dem Karpulenverschluss 26 zugewendeten Seite einen die Kanüle 20 in einer vorbestimmbaren radialen Distanz vollständig umschließenden und in axialer Richtung vorspringenden Dichtansatz 85 umfasst. Dieser ist im Axialschnitt gesehen keilförmig verjüngend hin in Richtung auf den Karpulenverschluss 26 ausgebildet. Beim Durchstechen des Karpulenverschlusses 26 durch die Kanüle 20 kann es zu Undichtheiten im unmittelbaren Durchstichbereich zwischen der Kanüle 20 und dem Karpulenverschluss 26 kommen. Die Dichtheit wird dann durch den Dichtansatz 85 im Zusammenwirken mit dem elastisch verformbar ausgebildeten Karpulenverschluss 26 hergestellt, da der Dichteinsatz 85 in das Material eindringt und dieses zusätzlich auch noch an die Kanüle 20 andrückt.

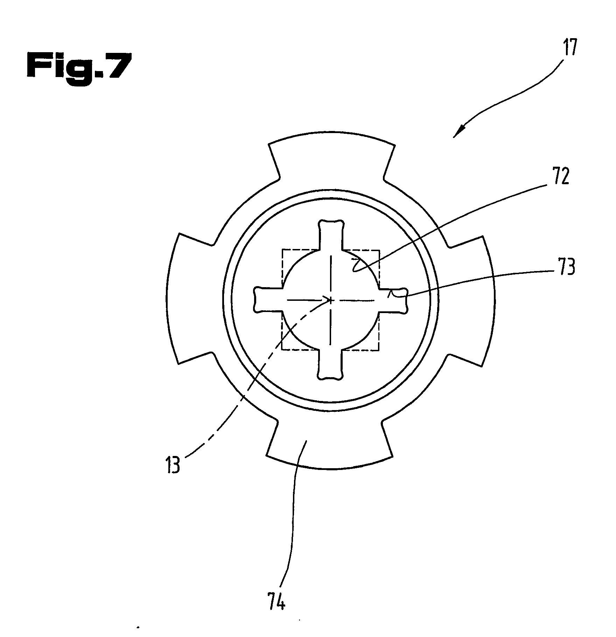

In der Fig. 7 ist die Haltescheibe 17 der Sicherungsvorrichtung 5 in Draufsicht und vereinfachter Darstellung gezeigt. So weist die Haltescheibe 17 in ihrem Zentrum einen bevorzugt kreisrunden Durchbruch 72 auf sowie zusätzlich daran anschließend sowie sich radial nach außen erstreckende Ausnehmungen 73. Unabhängig davon wäre es aber auch möglich, den Querschnitt des Durchbruchs 72 mehreckig, insbesondere quadratisch, auszubilden, wie dies in strichlierten Linien angedeutet ist. Die Anzahl der Ausnehmungen 73 ist dabei korrespondierend zu der Anzahl der in der Aufbewahrungsstellung festzulegenden Haltearme 16 der ersten Antriebsbaugruppe 4 zu wählen. Im vorliegenden Ausführungsbeispiel sind vier Haltearme 16 gleichmäßig über den Umfang verteilt vorgesehen, wobei zur besseren Positionierung, Führung sowie Halterung der Haltearme 16 in der Haltescheibe 17 die Ausnehmungen 73 vorgesehen sind. Weiters weist die Haltescheibe 17 an ihrem äußeren Umfang zusätzliche segmentartige Ansätze 74 auf. Diese Ansätze 74 dienen zur Verbindung der Haltescheibe 17 mit dem hinteren Traggehäuseteil 28 des Traggehäuses 2. Dies kann beispielsweise in Form einer Schnapp- oder Rastverbindung erfolgen.

Die Abgabe des in der Karpule 3 bevorrateten Medikaments 14 kann folgendermaßen durchgeführt werden. Die beiden Antriebsbaugruppen 4, 8 sind in ihrer vorgespannten Stellung innerhalb der Injektionsvorrichtung 1 gesichert gehalten, wobei der vordere Aufnahmeraum 29 mitsamt der gesamten Nadelanordnung 6 sich in sterilem Zustand befindet. Soll nun die Injektion verabreicht werden, ist zuerst durch Abnahme der Sicherungsvorrichtung 5, nämlich der Sicherungskappe 18 mitsamt dem Sicherungsstift 19, die erste Antriebsbaugruppe 4 für deren Auslösung bzw. Aktivierung freizugeben. Der Benutzer hält die gesamte Injektionsvorrichtung 1 an der Aktivierungshülse 9 fest und setzt diese mit ihrem distalen Ende 11 an der die Medizin bzw. das Medikament 14 zu verabreichenden Körperstelle des Patienten 10 an. Dadurch erfolgt eine Abstützung des Traggehäuses 2 mitsamt dem daran angeordneten Nadelschutzelement 7 an der Oberfläche des Patienten 10.

Durch die axiale Verstellbewegung der Aktivierungshülse 9 hin in Richtung auf das distale Ende 11 wird die erste Antriebsbaugruppe 4 ausgelöst bzw. aktiviert, wodurch die Karpule 3 mitsamt der Nadelanordnung 6 automatisch hin in Richtung auf das distale Ende 11 verstellt wird. Während dieser axialen Verstellbewegung wird zusätzlich noch der Nadelträger 23 mitsamt der Kanüle 20 von der verrasteten ersten Stellung am Führungselement 24 gelöst und das Nadelende 22 durch den Karpulenverschluss 26 hindurch gestochen. Vor diesem Hindurchstechen ist bereits das dem distalen Ende 11 zugewendete Nadelende 21 in den Patienten 10 eingedrungen, wobei die Strömungsverbindung zwischen dem davon abgewendeten Nadelende 22 mit dem Innenraum der Karpule 3 unmittelbar nachfolgend erfolgt. Unmittelbar danach oder auch gleichzeitig mit der axialen Verstellung der Aktivierungshülse 9 wird auch die zweite Antriebsbaugruppe 8 für die Bewegung des Nadelschutzelements 7 ausgelöst. Durch die erste Antriebsbaugruppe 4 wird dann das Medikament 14 an den Patienten 10 abgegeben. Nach Beendigung der Abgabe wird die gesamt Injektionsvorrichtung 1 vom Patienten 10 entfernt. Durch die bereits aktivierte bzw. ausgelöste zweite Antriebsbaugruppe 8 wird während der Entfernung der Injektionsvorrichtung 1 vom Patienten 10 das Nadelschutzelement 7 solange verstellt, bis dass die Kanüle 20 vollständig darin aufgenommen ist bzw. der Rastteil 71 des Nadelschutzelements 7 am Stellelement 70 zur Anlage kommt. Gleichzeitig mit der vollständigen axialen Verstellung des Nadelschutzelements 7 in die abdeckende Stellung wird dieses mittels dem Sicherungselement 69 durch Abstützten an der Anschlagfläche 57 gegen eine erneute Rückstellung in eine Freigabeposition des Nadelendes 21 gehindert.

In den Fig. 8 bis 10 ist eine weitere und gegebenenfalls für sich eigenständige Ausführungsform einer Aufnahmeschale 75 zur Aufnahme der Injektionsvorrichtung 1 bzw. einer Mehrzahl dieser gezeigt, wobei wiederum für gleiche Teile gleiche Bezugszeichen bzw. Bauteilbezeichnungen wie in den vorangegangenen Fig. 1 bis 7 verwendet werden. Um unnötige Wiederholungen zu vermeiden, wird auf die detaillierte Beschreibung in den vorangegangenen Fig. 1 bis 7 hingewiesen bzw. Bezug genommen.

Die hier gezeigte Aufnahmeschale 75 kann für sich eine eigenständige erfindungsgemäße Ausbildung darstellen. Dabei kann die Aufgabe der Erfindung darin gesehen werden, eine Aufnahmeschale 75 zu schaffen, welche zur Aufnahme von bevorzugt mehreren nebeneinander angeordneten Injektionsvorrichtungen 1 dient und in welcher auch eine Sterilisierung der Injektionsvorrichtung 1 erfolgen kann, wobei nach der Sterilisierung der nachfolgende Verschlussvorgang und der damit verbundene sterile Verschluss des sterilisierten Raumes innerhalb der Injektionsvorrichtung 1 auch gleichzeitig noch bei in der Aufnahmeschale 75 befindlicher Injektionsvorrichtung 1 durchgeführt werden kann.

Die Aufnahmeschale 75 kann dabei auch als Tray bezeichnet werden, welches beispielsweise aus einer tiefgezogenen Folie aus einem wärmeverformbaren Material, wie beispielsweise aus einem Kunststoff, gebildet ist. Die in den Fig. 8 und 9 dargestellte Injektionsvorrichtung 1 in der Aufnahmeschale 75 ist ungeschnitten und nur vereinfacht schematisiert dargestellt.

Die Aufnahmeschale 75 ist wannenförmig ausgebildet und weist einen Boden 76 sowie von diesem aufragende Seitenwände 77 auf. Auf der vom Boden 76 abgewendeten Seite der Seitenwände 77 weist die Aufnahmeschale 75 einen Behälterrand 78 auf. Dieser ist bevorzugt flanschförmig nach außen ragend ausgebildet und dient dazu, um mit einer in der Fig. 8 schematisch vereinfacht dargestellten Abdeckung 79 verschlossen zu werden. Die Abdeckung 79 deckt den gesamten zur Aufnahme der Injektionsvorrichtung 1 vorgesehenen und von der Aufnahmenschale 75 umgrenzten Innenraum ab und kann aus dem zuvor beschriebenen Material bzw. Werkstoff gewählt werden, wie dieser für die Umhüllung der Injektionsvorrichtung 1 beschrieben worden ist. Da das papierfließartige Faserfunktionstextil aus den thermisch verschweißten Fasern einen Siegel- bzw. Verschweißvorgang mit dem Grundmaterial der Aufnahmeschale 75 bei entsprechender Wahl zulässt, kann im Bereich des Behälterrandes 78 die Abdeckung 79 auf diese Art und Weise rundum durchlaufend dichtend befestigt sein. Es wäre aber auch möglich, die Abdeckung 79 durch eine dichte Klebeverbindung oder ähnliches am Behälterrand 78 der Aufnahmeschale 75 zu haltern. Wesentlich bei dem Werkstoff der Abdeckung 79 ist, dass es möglich ist, die Begasung und die damit einhergehende Sterilisation durch die Abdeckung 79 hindurch durchführen zu können, ohne dass nachträglich noch Keime bzw. Bakterien durch die Abdeckung 79 hindurch treten können. Der Werkstoff der Aufnahmeschale 75 ist dann auch so zu wählen, dass hier ebenfalls ein vollständiger keim - bzw. bakteriendichter Verschluss erfolgt.

In Abhängigkeit von den Außenabmessungen der Aktivierungshülse 9 sind hier im Bereich des Bodens 76 Halteelemente 80 in hintereinander angeordneten Reihen im Bereich des Bodens 76 ausgeformt. Diese dienen zur klemmenden Aufnahme der Injektionsvorrichtung 1 und legen somit die Position der Injektionsvorrichtungen 1 innerhalb der Aufnahmeschale 75 fest. Es wäre aber auch möglich, auf die klemmende Wirkung der Halteelemente 80 zu verzichten und diese gänzlich weg zu lassen. Unabhängig davon wäre es auch noch denkbar, anstatt der klemmenden Wirkung der Halteelemente 80 zwischen den in der Aufnahmeschale 75 angeordneten Injektionsvorrichtungen 1 lediglich Trennstege anzuordnen, um so zumindest eine grobe Positionierung bzw. gegenseitige Lageausrichtung der Injektionsvorrichtungen 1 zueinander zu erzielen.

Weiters ist aus einer Zusammenschau der Fig. 8 bis 10 im Bereich des distalen bzw. proximalen Endes 11, 12 der Injektionsvorrichtung 1 zu ersehen, dass an den gegenüberliegenden Seitenwänden 77 jeweils verformbare Wandabschnitte 81 angeordnet bzw. ausgebildet sind. Diese einzelnen Wandabschnitte 81 sind dabei jeweils dem distalen Ende 11 bzw. proximalen Ende 12 unmittelbar benachbart zugeordnet. In jener Kupplungsstellung der beiden Traggehäuseteile 27 und 28, bei welcher ein Zugang durch die Durchsetzung 37 in den vorderen Aufnahmeraum 29 möglich ist, ragen bevorzugt senkrecht zum Boden 76 ausgerichtete Stützabschnitte 82 der verformbaren Wandabschnitte 81 bis unmittelbar an das distale bzw. proximale Ende 11, 12 heran. Bei entsprechender Maßwahl wird hier eine ausreichende axiale Ausrichtung der einzelnen Injektionsvorrichtungen 1 innerhalb der Aufnahmeschale 75 erzielt. Es wäre aber unabhängig davon möglich, auf die verformbaren Wandabschnitte 81 zu verzichten und den oder die Stützabschnitte 82 direkt durch die Seitenwände 77 in nahezu ebenflächiger Ausbildung auszubilden. Dabei ist eine ausreichende Möglichkeit der Verformungsbewegung der Seitenwände 77 zur Durchführung der zuvor beschriebenen Verstellbewegung der beiden Traggehäuseteile 27, 28 relativ zueinander sicher zu stellen.

Anschließend an diesen Stützabschnitt 82 umfasst der verformbare Wandabschnitt 81 stufenförmig nach außen erweiternde und jeweils halbkreisförmig verlaufende Verformungsabschnitte 83. Die stufenförmig hintereinander angeordneten Verformungsabschnitte 83 schließen sich dabei anschließend an den Stützabschnitt 82 hin in Richtung des Behälterrandes 87 daran an. Der Beginn der halbkreisförmig verlaufenden Verformungsabschnitte 83 ist dabei in etwa in der Höhe der Längsachse 13 der Injektionsvorrichtung 1 bei in der Aufnahmeschale 75 geklemmter Stellung sowie bevorzugt parallel zum Boden 76 bzw. dem Behälterrand 78 gewählt. Durch die treppenförmige bzw. stufenförmige hintereinander Anordnung wird ein sehr hoher Verformungsweg der Wandabschnitte 81 erzielt, ohne dass bei diesen Verformungswegen eine Beschädigung der Aufnahmeschale 75 mit einher geht. Dies ist deshalb wesentlich, da ansonsten der Eintritt von Keimen bzw. Bakterien in den ansonst rundum abgeschlossenen Aufnahmeraum der Aufnahmeschale 75 ermöglich wird.

In der Fig. 8 ist vereinfacht mit aufeinander zu gerichteten Pfeilen mit der Kennung "F" zu ersehen, dass durch die Aufbringung der eingezeichneten Druckkraft die zuvor beschriebene Verstellbewegung zwischen den beiden Traggehäuseteilen 27, 28 bewirkt werden kann.

Weiters ist in Fig. 8 noch vereinfacht dargestellt, dass die Injektionsvorrichtung 1 eine Markierung 84 aufweisen kann, welche in der ersten Kupplungsstellung der beiden Traggehäuseteile 27, 28 zu ersehen ist, jedoch in der in der Fig. 9 dargestellten zweiten Kupplungsstellung nicht mehr erkennbar bzw. sichtbar ist. Unabhängig davon wären aber auch andere Möglichkeiten denkbar, um den ordnungsgemäßen Abschluss des vorderen Aufnahmeraums 29 optisch erkennbar darzustellen, ohne dass dazu komplizierte Überwachungseinrichtungen notwendig sind. Dies wäre beispielsweise durch eine einfache rot/ grün Markierung in einem Sichtfenster in der Aktivierungshülse 9 möglich, wobei beispielsweise die rote Markierung die erste Kupplungsstellung und die grüne Markierung die zweite, sterile Kupplungsstellung zwischen den beiden Traggehäuseteilen 27, 28 anzeigen kann. Dies kann durch die relative Stellung des vorderen Traggehäuseteils 27 bezüglich der Aktivierungshülse 9 erfolgen, wobei die angezeigte Markierung von der jeweiligen Stellung abhängig ist.

Durch die zusätzliche Ausbildung bzw. Verwendung der Aufnahmeschale 75 wird es möglich, die Injektionsvorrichtung 1 in einem vormontierten Zustand für die Sterilisation bereit zu stellen, um diese dann beispielsweise an einem dafür vorgesehenen weiteren Betriebsstandort durchführen zu können und nach erfolgter Sterilisation die Verstellung der beiden Traggehäuseteile 27, 28 in die zweite Kupplungsstellung durchzuführen. Durch das vollständige Umschließen der Injektionsvorrichtungen 1 innerhalb der Aufnahmeschale 75 und der damit verbundenen Abdeckung 79 können auch nicht sterile Transportwege nach der Sterilisation bis hin zum vollständigen Verschluss in die zweite Kupplungsstellung eingeplant werden.

Anschließend an die durchgeführte Sterilisation sind somit alle durch die ETO-Begasung zugänglichen Bauteile der Injektionsvorrichtung 1 sterilisiert bzw. weisen diese einen derartigen Zustand auf. Das nachträgliche Verstellen der beiden Traggehäuseteilen 27, 28 gegeneinander und dem damit verbundenen vollständig dichten Abschluss des vorderen Aufnahmeraums 29 kann dann mittels einer eigenen maschinellen, hier nicht näher dargestellten Einrichtung durchgeführt werden. Der ordnungsgemäße Abschluss kann durch die zuvor beschriebene optische oder sensorische Kontrolle der Markierung 84 erfolgen.

Nach erfolgtem Zusammenschieben der beiden Traggehäuseteile 27, 28, kann die Abdeckung 79 von der Aufnahmeschale 75 entfernt und die sterilisierten Injektionsvorrichtungen 1 aus der Aufnahmeschale 75 entnommen und gegebenenfalls in Einzelverpackungen weiterverpackt werden.

In den Fig. 11 und 12 ist eine weitere und gegebenenfalls für sich eigenständige Ausführungsform der Nadelanordnung 6, insbesondere des Nadelhalters 23 und des Führungselements 24, der Injektionsvorrichtung 1 gezeigt, wobei wiederum für gleiche Teile gleiche Bezugszeichen bzw. Bauteilbezeichnungen wie in den vorangegangenen Fig. 1 bis 7 verwendet werden. Um unnötige Wiederholungen zu vermeiden, wird auf die detaillierte Beschreibung in den vorangegangenen Fig. 1 bis 7 hingewiesen bzw. Bezug genommen.

Wie bereits zuvor beschrieben, ist der Nadelträger 23 und die darin aufgenommene bzw. gehalterte Kanüle 20 gemeinsam in Axialrichtung längs verschieblich bezüglich dem Führungselement 24 in diesem gelagert. In der Fig. 11 ist weiters noch die Abstützung des Halteelements 63 am Führungselement 24 zu ersehen. An dem Halteelement 63 ist die zuvor beschriebene Stellnocke 64 angeordnet, welche bei Axialverstellung der Karpule 3 mitsamt dem Führungselement 24 sowie dem Nadelträger 23 die Arretierung löst und dabei der Einstichvorgang des Nadelendes 22 in den Karpulenverschluss 26 erfolgt.

Das Führungselement 24 ist bei diesem hier gezeigten Ausführungsbeispiel aus zwei Führungsteilen 87, 88 gebildet, welche halbschalenförmig zu dem rohrförmigen Führungselement 24 zusammengefügt werden können.

Durch die in der Fig. 12 dargestellte abgehobene Stellung des hier oben liegenden Führungsteils 87 ist die distanzierte Stellung des Nadelendes 22 vom Karpulenverschluss 26 gut zu ersehen. Weiters weist der Nadelträger 23 bevorzugt diametral gegenüberliegende Führungsansätze 89 auf, welche in einen Führungsschlitz 90 im Führungsteil 87 und/oder 88 eingreifen.

In der in der Fig. 11 dargestellten und montierten Stellung des Führungselements 24 ist der Nadelträger 23 in beiden Axialrichtungen fixiert relativ gegenüber dem Führungselement 24 gehaltert. Der oder die Führungsansätze 89 begrenzen im Zusammenwirken mit dem Führungsschlitz 90 die Verstellbewegung des Nadelträgers 23 hin in Richtung auf das distale Ende 11. Das oder die Halteelemente 63 hingegen verhindern eine Axialverstellung des Nadelträgers 23 hin in Richtung auf das proximale Ende 12 bis zu deren Freigabe durch das Zusammenwirken der Stellnocke 64 mit der Steuerkurve 66 des Traggehäuses 2. Die relative axiale Verstellbewegung des Nadelträgers 23 hin in Richtung auf das proximale Ende 12 und dem damit verbundenen Durchstechen des Nadelendes 22 durch den Karpulenverschluss 26 kann durch die gewählte Längserstreckung des Führungsschlitzes 90 begrenzt werden.

Die beiden hier als Halbschalenteile ausgebildeten Führungsteile 87, 88 werden im zusammengebauten Zustand durch die Anlage derselben an der Innenfläche 45 des Traggehäuses 2 insbesondere des vorderen Traggehäuseteils 27 an einem Auseinanderfallen gehindert. Dadurch wird eine einfache Montage bei gutem Zusammenhalt erzielt. Darüber hinaus ist auch eine gegenseitige Arretierung der beiden Führungsteile 87, 88 durch zueinander versetzt angeordnete Zapfen und Zapfenaufnahmen möglich.

Diese axiale Führung hat den Vorteil, dass auch eine axiale Einstichbewegung der Kanüle 20 bezüglich der Längsachse 13 in das Gewebe des Patienten 10 oder des Anwenders erfolgt.

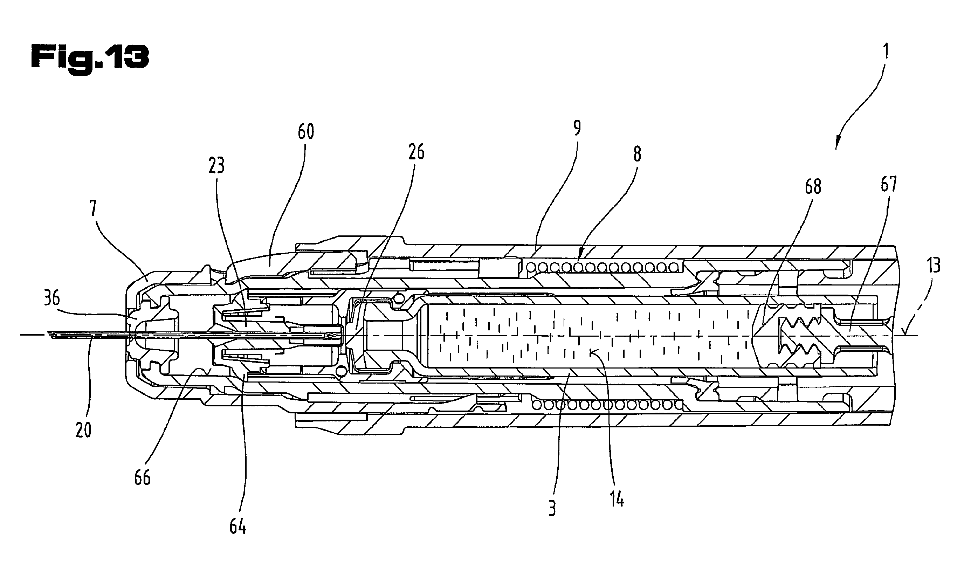

In der Fig. 13 ist das bereits zuvor detailliert beschriebene Zusammenwirken der Steuerkurve 66 des Traggehäuses 2, im vorliegenden Ausführungsbeispiel des vorderen Traggehäuseteils 27, mit der am Halteelement 63 angeordneten Stellnocke 64 in vergrößerter Darstellung gezeigt.

Dies führt zur radialen Verlagerung und dem damit verbundenen Lösen der Arretierung zwischen dem Halteelement 63 und dem Führungselement 24. In Abhängigkeit der Lage der Steuerkurve 66 in axialer Richtung gesehen kann auch die Freigabe zur Einstichbewegung des Nadelendes 22 in den Karpulenstopfen 26 festgelegt werden. Je früher diese Freigabe erfolgt, desto eher steht die Kanüle 20 mit dem das Medikament 14 aufnehmenden Innenraum der Karpule 3 in Strömungsverbindung.

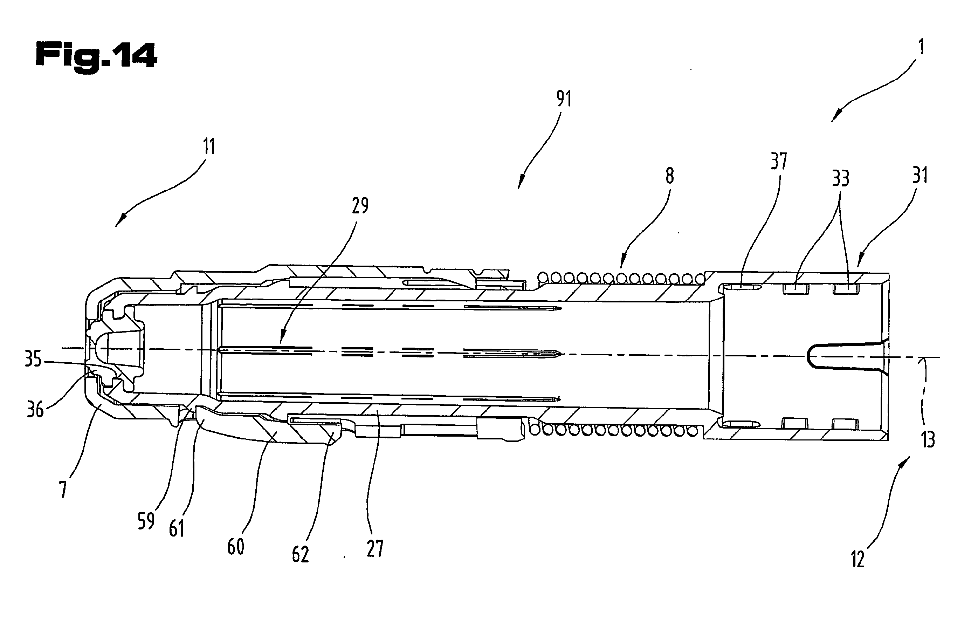

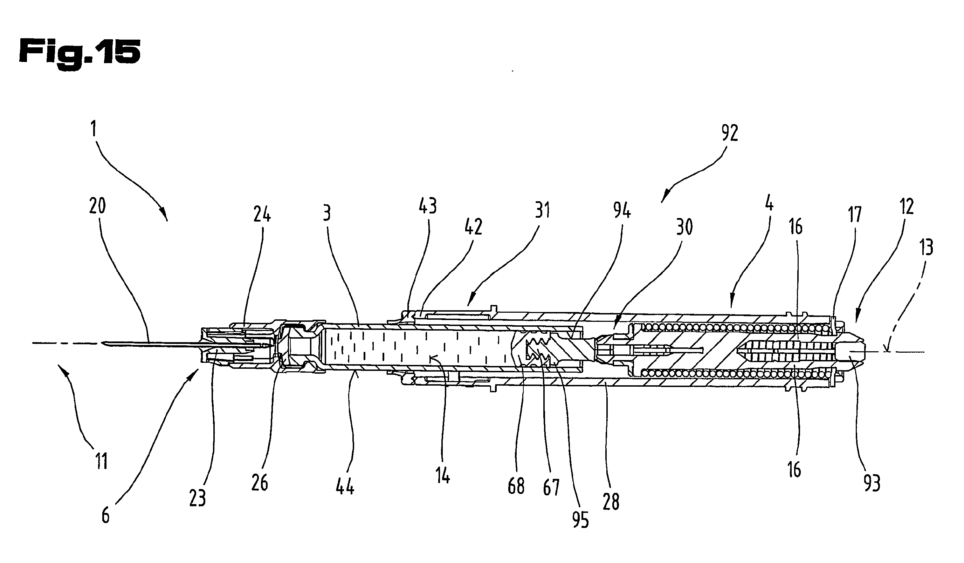

In den Fig. 14 und 15 ist die Injektionsvorrichtung 1 gemäß der Darstellung in den Fig. 1 bis 7 sowie 11 bis 13 gezeigt, wobei wiederum für gleiche Teile gleiche Bezugszeichen bzw. Bauteilbezeichnungen wie in den vorangegangenen Figuren verwendet werden. Um unnötige Wiederholungen zu vermeiden, wird auf die detaillierte Beschreibung in den vorangegangenen Fig. 1 bis 13 hingewiesen bzw. Bezug genommen.

Die die Injektionsvorrichtung 1 bildenden einzelnen Bauteile sind derart ausgelegt, dass diese zu Baugfuppen bzw. Baueinheiten 91, 92 zusammenstellbar bzw. vormontierbar sind. In einem nachfolgenden weiteren Montageschritt werden dann die beiden Baueinheiten 91, 92 mit den noch verbleibenden Bauteilen zusammengefügt wie dies in der Fig. 1 dargestellt ist.

So umfasst eine erste Baueinheit 91 im Bereich des distalen Endes 11 der Injektionsvorrichtung 1 den vorderen Traggehäuseteil 27, den Dichtstopfen 36 sowie das mit der zweiten Antriebsbaugruppe 8 verstellbare Nadelschutzelement 7. Das Nadelschutzelement 7 ist seinerseits über das zuvor beschriebene Hebelelement 60 am Anschlagelement 59 des vorderen Traggehäuseteils 27 abgestützt und verrastet gehalten. Die zweite Antriebsbaugruppe 8, welche hier durch eine spiralförmig ausgebildete Druckfeder gebildet ist, stützt sich einerseits an dem stufenförmig abgesetzten Verbindungsabschnitt 31 des vorderen Traggehäuseteils 27 und andererseits an einer dem proximalen Ende 12 zugewendeten Stirnseite des Nadelschutzelements 7 ab. Durch die vorgespannte Antriebsbaugruppe 8 kann nach der bereits zuvor beschriebenen Auslösung des Hebelelements 60 die Axialverstellung des Nadelschutzelements 7 in die die Nadel bzw. die Kanüle 20 abdeckende Stellung erfolgen.

Die zweite Baueinheit 92 im Bereich des proximalen Endes 12 der Injektionsvorrichtung 1 umfasst ihrerseits das hintere Traggehäuseteil 28, die darin aufgenommene erste Antriebsbaugruppe 4, die Karpule 3 mit ihrem Karpulenstopfen 26, die Nadelanordnung 6, das Dichtelement 43, die Kolbenstange 67 mit dem Karpulenstopfen 68 sowie die Haltearme 16, welche mittels der Haltescheibe 17 positioniert gehalten werden können. Um ein ungewolltes Auslösen der ersten Antriebsbaugruppe 4 durch das außer Eingriff kommen der Haltearme 16 mit der Haltescheibe 17 zu vermeiden, ist hier in der Fig. 15 noch vereinfacht dargestellt, dass die Haltearme 16 über ein im Bereich der Längsachse 13 angeordnetes Sicherungselement 93 radial auf die von der Längsachse 13 abgewendete Seite gehalten sind. Das Sicherungselement 93 ist bevorzugt stiftförmig ausgebildet und wird so lange an der Baueinheit 92 belassen, bis dass der endgültige Zusammenbau mit der Baueinheit 91 sowie der Aktivierungshülse 11 und der Sicherungsvorrichtung 5 erfolgt.

Dadurch kann ein hoher Grad an Vorfertigung erfolgen, wobei je nach gewünschtem Medikament auch noch die Karpule 3 mit der daran angeordneten Nadelanordnung 6 über den Karpulenstopfen 68 mit der Kolbenstange 67 verbunden werden kann. Die Montage der Karpule 3 an der Kolbenstange 67 kann dann in Abhängigkeit von der Lagerfähigkeit des Medikaments 14 erfolgen.

Damit kann ein einfaches Verfahren zum Zusammenbau der Injektionsvorrichtung 1 geschaffen werden, bei dem zuerst die zuvor beschriebenen Baueinheiten 91, 92 mit all ihren Einzelteilen zusammengebaut werden und so ein Halbfabrikat bzw. Vorprodukt bilden. Damit kann auch eine externe Fertigung der einzelnen Bauteile erfolgen und beispielsweise von unterschiedlichen Herstellern die vormontierten Baueinheiten 91, 92 getrennt voneinander gefertigt und zusammengebaut werden. Wird im Zuge des Zusammenbaus die erste Baueinheit 91 mit der zweiten Baueinheit 92 im Bereich des Verbindungsabschnitts 31 gemäß der in der Fig. 1 dargestellten Position zusammengefügt, ist dann diese Baugruppe aus den beiden Baueinheiten 91, 92 in die Aktivierungshülse 9 einzusetzen. Bereits zuvor oder aber auch im Anschluss daran ist das bevorzugt stiftförmig ausgebildete Sicherungselement 93 im Bereich der Haltearme 16 zu entfernen und durch die kappenförmig ausgebildete Sicherungsvorrichtung 5 mit dem Sicherungsstift 19 zu ersetzen. Anschließend daran kann die zuvor beschriebene Sterilisation im Innenraum des Traggehäuses 2, insbesondere im Bereich der Nadelanordnung 6 erfolgen.

Wie bereits auch zuvor in den Fig. 1 bis 6 dargestellt, weist die Kolbenstange 67 eine radial umlaufende Vertiefung 94 auf, welche in der vormontierten Stellung der Kolbenstange 67 an der Karpule 3 an dem dem proximalen Ende 12 zugewendeten Ende der Karpule 3 zu liegen kommt bzw. angeordnet ist. Diese Reduzierung der äußeren Abmessung, insbesondere des Durchmessers der Kolbenstange 67 dient dazu, um während der gesamten Lagerdauer der Injektionsvorrichtung bei einem möglichen Verschwenken der Karpule 3 relativ bezüglich der Kolbenstange 67 eine Beschädigung der zumeist oder bevorzugt aus Glas gebildeten Karpule 3 an ihrem proximalen Ende zu vermeiden. So wird die Kolbenstange 67 mit dem Karpulenstopfen 68 verbunden. Dies kann bevorzugt durch eine Gewindeanordnung erfolgen. Im unmittelbaren Anlagebereich der Kolbenstange 67 am Karpulenstopfen weist diese einen bevorzugt flanschförmig ausgebildeten Ansatz 95 auf. Dieser Ansatz 95 dient zur gegenseitigen axialen Fixierung zwischen Kolbenstange 67 und Karpulenstopfen 68. Die als Vertiefung 94 ausgebildete Freistellung der Kolbenstange 67 schützt somit vor Beschädigung während des Transports bzw. bei einem unbeabsichtigten Hinunterfallen der gesamten Injektionsvorrichtung 1. Eine weitere dämpfende Abstützung der Karpule 3 kann über das Dichtelement 43 am hinteren Traggehäuseteil 28 erfolgen, wie dies bereits zuvor beschrieben worden ist.

Die Ausführungsbeispiele zeigen mögliche Ausführungsvarianten der Injektionsvorrichtung 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Injektionsvorrichtung 1 diese bzw. deren Bestandteile teilweise unmaßstäblich und/oder vergrößert und/oder verkleinert dargestellt wurden.

Die den eigenständigen erfinderischen Lösungen zugrunde liegende Aufgabe kann der Beschreibung entnommen werden.

Vor allem können die einzelnen in den Fig. 1 bis 6; 7; 8 bis 10; 11, 12; 13; 14; 15 gezeigten Ausführungen jeweils den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

**Bezugszeichenaufstellung**

| | | | |
|---|---|---|---|
| 1 | Injektionsvorrichtung | 41 | Ansatz |
| 2 | Traggehäuse | 42 | Ende |
| 3 | Karpule | 43 | Dichtelement |
| 4 | Antriebsbaugruppe | 44 | Außenfläche |
| 5 | Sicherungsvorrichtung | 45 | Innenfläche |
| | | | |
| 6 | Nadelanordnung | 46 | Mantel |
| 7 | Nadelschutzelement | 47 | Hülsenwand |
| 8 | Antriebsbaugruppe | 48 | Aufnahmebereich |
| 9 | Aktivierungshülse | 49 | Kappenmantel |
| 10 | Patient | 50 | Aktivierungselement |
| | | | |
| 11 | distales Ende | 51 | Kegelfläche |
| 12 | proximales Ende | 52 | Rastvorrichtung |
| 13 | Längsachse | 53 | Rastnase |
| 14 | Medikament | 54 | Rastnase |
| 15 | Antriebsmittel | 55 | Rastelement |
| | | | |
| 16 | Haltearm | 56 | Innenfläche |
| 17 | Haltescheibe | 57 | Anschlagfläche |
| 18 | Sicherungskappe | 58 | Oberfläche |
| 19 | Sicherungsstift | 59 | Anschlagelement |
| 20 | Kanüle | 60 | Hebelelement |
| | | | |
| 21 | Nadelende | 61 | Hebelende |
| 22 | Nadelende | 62 | Hebelende |
| 23 | Nadelträger | 63 | Halteelement |
| 24 | Führungselement | 64 | Stellnocke |
| 25 | Karpulenende | 65 | Innenwand |
| | | | |
| 26 | Karpulenverschluss | 66 | Steuerkurve |
| 27 | Traggehäuseteil | 67 | Kolbenstange |
| 28 | Traggehäuseteil | 68 | Karpulenstopfen |
| 29 | Aufnahmeraum | 69 | Sicherungselement |
| 30 | Aufnahmeraum | 70 | Stellelement |
| | | | |
| 31 | Verbindungsabschnitt | 71 | Rastteil |
| 32 | Kupplungsvorrichtung | 72 | Durchbruch |
| 33 | Arretierelement | 73 | Ausnehmung |
| 34 | Arretierelement | 74 | Ansatz |
| 35 | Öffnung | 75 | Aufnahmeschale |
| | | | |
| 36 | Dichtstopfen | 76 | Boden |
| 37 | Durchsetzung | 77 | Seitenwand |
| 38 | Wandteil | 78 | Behälterrand |
| 39 | Stirnwand | 79 | Abdeckung |
| 40 | Antriebsmittel | 80 | Halteelement |
| 81 | Wandabschnitt | | |
| 82 | Stützabschnitt | | |
| 83 | Verformungsabschnitt | | |
| 84 | Markierung | | |
| 85 | Dichtansatz | | |
| | | | |
| 86 | Dämpfungsvorrichtung | | |
| 87 | Führungsteil | | |
| 88 | Führungsteil | | |
| 89 | Führungsansatz | | |
| 90 | Führungsschlitz | | |
| | | | |
| 91 | Baueinheit | | |
| 92 | Baueinheit | | |
| 93 | Sicherungselement | | |
| 94 | Vertiefung | | |
| 95 | Ansatz | | |

## Patentansprüche

1. Injektionsvorrichtung (1), insbesondere Autoinjektor, der von einer Aufbewahrungsstellung in eine Injektionsstellung verstellbar ausgebildet ist, mit einem Traggehäuse (2), das ein einem Patienten (10) zuwendbares distales Ende (11) und ein davon abgewendetes proximales Ende (12) aufweist, zwischen welchen sich eine Längsachse (13) erstreckt,
einer Aktivierungshülse (9), die das Traggehäuse (2) zumindest teilweise umgibt, wobei das distale Ende (11) des Traggehäuses (2) die Aktivierungshülse (9) überragt und das Traggehäuse (2) über den größten Teil seiner Längserstreckung zwischen dem distalen und dem proximalen Ende (11, 12) von der Aktivierungshülse (9) umgeben ist, wobei die Aktivierungshülse (9) relativ bezüglich des Traggehäuses (2) in axialer Richtung verstellbar ist,
einer Karpule (3) mit einem darin enthaltenen und beim Injektionsvorgang abzugebenden Medikament (14), wobei die Karpule (3) im Traggehäuse (2) aufgenommen ist,
einer ersten Antriebsbaugruppe (4), die mit der Karpule (3) sowie mit der Aktivierungshülse (9) in Wirkverbindung steht und mittels der Aktivierungshülse (9) auslösbar ist,
einer Sicherungsvorrichtung (5), die die erste Antriebsbaugruppe (4) bis zu deren Aktivierung durch die Aktivierungshülse (9) für den Injektionsvorgang bezüglich des Traggehäuses (2) in ihrer Position festlegt,
einer Nadelanordnung (6), die der Karpule (3) in der Aufbewahrungsstellung der Injektionsvorrichtung (1) im Abschnitt des distalen Endes (11) vorgeordnet ist sowie beide Nadelenden (21, 22) einer Kanüle (20) innerhalb des Traggehäuses (2) angeordnet sind,
einem Nadelschutzelement (7), das von einer nicht wirksamen Stellung in eine das nach dem Injektionsvorgang über das distale Ende (11) des Traggehäuses (2) vorragende Nadelende (21) abdeckende Stellung verlagerbar ist,
einer zweiten auslösbaren Antriebsbaugruppe (8), die das Nadelschutzelement (7) von der nicht wirksamen Stellung in die abdeckende Stellung verlagert,
**dadurch gekennzeichnet, dass**
das Nadelschutzelement (7) mit der dieser zusammenwirkenden zweiten Antriebsbaugruppe (8) im Axialschnitt gesehen radial zwischen dem Traggehäuse (2) und der Aktivierungshülse (9) angeordnet ist und dass das Traggehäuse (2) einen vorderen und einen hinteren Traggehäuseteil (27, 28) mit jeweils einem vorderen und hinteren Aufnahmeraum (29, 30) umfasst, und die beiden Traggehäuseteile (27, 28) in einem Verbindungsabschnitt (31) über eine Kupplungsvorrichtung (32) in Richtung der Längsachse (13) in zwei unterschiedlichen Längspositionen in einer ersten und einer zweiten Kupplungsstellung miteinander kuppelbar sind.

2. Injektionsvorrichtung nach Anspruch 1, **dadurch gekennzeichnet, dass** im Verbindungsabschnitt (31) der Kupplungsvorrichtung (32) der vordere Traggehäuseteil (27) den hinteren Traggehäuseteil (28) übergreift.

3. Injektionsvorrichtung nach einem der Ansprüche 1 oder 2, **dadurch gekennzeichnet, dass** die Kupplungsvorrichtung (32) an den beiden Traggehäuseteilen (27, 28) jeweils zusammenwirkende Arretierelemente (33, 34) umfasst und diese derart ausgebildet sind, dass die beiden Traggehäuseteile (27, 28) jeweils in den beiden Kupplungsstellungen in einer in Richtung der Längsachse (13) gesehen gegensinnigen und voneinander entfernenden Bewegungsrichtung arretiert sind.

4. Injektionsvorrichtung nach einem der Ansprüche 1 bis 3, **dadurch gekennzeichnet, dass** das distale Ende (11) des vorderen Traggehäuseteils (27) eine den Durchtritt des Nadelendes (21) der Nadelanordnung (6) ermöglichende Öffnung (35) aufweist, in welcher ein durchstechbarer Dichtstopfen (36) in dichtender Stellung angeordnet ist.

5. Injektionsvorrichtung nach einem der Ansprüche 1 bis 4, **dadurch gekennzeichnet, dass** der vordere Traggehäuseteil (27) im Verbindungsabschnitt (31) eine Durchsetzung (37) aufweist.

6. Injektionsvorrichtung nach einem der Ansprüche 1 bis 5, **dadurch gekennzeichnet, dass** der vordere Traggehäuseteil (27) im Verbindungsabschnitt (31) an einer dem hinteren Traggehäuseteil (28) zugewendeten und in Richtung auf die Längsachse (13) rückspringenden Stirnwand (39) einen im Axialschnitt gesehen keilförmig ausgebildeten und über den Umfang durchlaufend ausgebildeten Ansatz (41) aufweist.

7. Injektionsvorrichtung nach einem der Ansprüche 1 bis 6, **dadurch gekennzeichnet, dass** am hinteren Traggehäuseteil (28) an einem dem vorderen Traggehäuseteil (27) zugewendeten Ende (42) ein über den Umfang durchlaufend ausgebildetes Dichtelement (43) angeordnet ist.

8. Injektionsvorrichtung nach Anspruch 7, **dadurch gekennzeichnet, dass** das Dichtelement (43) sowohl an einer Außenfläche (44) der Karpule (3) als auch einer Innenfläche (45) des vorderen Traggehäuseteils (27) dichtend anliegt und in der ersten Kupplungsstellung der beiden Traggehäuseteile (27, 28) distanziert von der Stirnwand (39) bzw. dem an der Stirnwand (39) ausgebildeten Ansatz (41) angeordnet ist.

9. Injektionsvorrichtung nach einem der Ansprüche 1 bis 8, **dadurch gekennzeichnet, dass** in der ersten Kupplungsstellung der beiden Traggehäuseteile (27, 28) die im vorderen Traggehäuseteil angeordnete Durchsetzung (37) in den vorderen Aufnahmeraum (29) des vorderen Traggehäuseteils (27) einmündet und mit diesem in Strömungsverbindung steht.

10. Injektionsvorrichtung nach einem der Ansprüche 7 oder 8, **dadurch gekennzeichnet, dass** in der zweiten Kupplungsstellung der beiden Traggehäuseteile (27, 28) das Dichtelement (43) dichtend an der rückspringenden Stirnwand (39) bzw. dem daran angeordneten Ansatz (41) anliegt und weiters der Zugang in den vorderen Aufnahmeraum (29) über die Durchsetzung (37) unterbrochen ist.

11. Injektionsvorrichtung nach einem der Ansprüche 1 bis 10, **dadurch gekennzeichnet, dass** der hintere Traggehäuseteil (28) an seiner äußeren Oberfläche und im Bereich seines proximalen Endes (12) zumindest zwei in Richtung der Längsachse (13) distanzierte Rastnasen (53, 54) aufweist, welche mit einem an der Aktivierungshülse (9) angeordneten Rastelement (55) derart zusammenwirken, dass die Rastnasen (53, 54) die relativen Stellungen des Traggehäuses (2) und der Aktivierungshülse (9) zueinander einerseits in der Aufbewahrungsstellung und andererseits in der Injektionsstellung definieren und dass ausschließlich eine relative Längsverstellung der Aktivierungshülse (9) bezüglich des Traggehäuses (2) von der Aufbewahrungsstellung hin zur Injektionsstellung ermöglicht ist, jedoch eine in entgegensetzter Richtung relative Längsverstellung verhindert ist.

12. Injektionsvorrichtung nach einem der vorhergehenden Ansprüche, **dadurch gekennzeichnet, dass** am Nadelschutzelement (7) ein wippenartig ausgebildetes Hebelelement (60) mit voneinander in Richtung der Längsachse (13) distanzierten Hebelenden (61, 62) angeordnet ist, wobei sich das Hebelelement (60) in der Aufbewahrungsstellung der Injektionsvorrichtung (1) mit seinem dem distalen Ende (11) des Traggehäuses (2) zugewendeten Hebelende (61) an einem am Traggehäuse (2) an dessen äußerer Oberfläche (58) angeordneten Anschlagelement (59) abstützt.

13. Injektionsvorrichtung nach einem der vorhergehenden Ansprüche, **dadurch gekennzeichnet, dass** die Aktivierungshülse (9) im Bereich des distalen Endes (11) sowie an deren Innenfläche (56) eine Anschlagfläche (57) aufweist, welche bevorzugt in senkrechter Richtung bezüglich der Längsachse (13) ausgerichtet ist, wobei das Nadelschutzelement (7) weiters an seinem proximalen Ende (12) ein elastisch verformbar ausgebildetes Sicherungselement (69) umfasst, welches bei der die Nadelanordnung (6) abdeckenden Stellung des Nadelschutzelements (7) an der an der Innenfläche (56) der Aktivierungshülse (9) ausgebildeten Anschlagfläche (57) abgestützt ist.

14. Injektionsvorrichtung nach einem der vorhergehenden Ansprüche, **dadurch gekennzeichnet, dass** das Sicherungselement (69) bei der die Nadelanordnung (6) abdeckenden Stellung des Nadelschutzelements (7) zusätzlich an einem am Traggehäuse (2) angeordneten Stellelement (70) radial daran abgestützt ist, wobei das Stellelement (70) durchgehend umlaufend ausgebildet ist und bei der die Nadelanordnung (6) abdeckenden Stellung des Nadelschutzelements (7) das Nadelschutzelement (7) mit seinem proximalen Ende (12) zusätzlich am Stellelement (70) in Richtung auf das distale Ende (11) des Traggehäuses (2) hin abgestützt ist.

15. Injektionsvorrichtung nach einem der vorhergehenden Ansprüche, **dadurch gekennzeichnet, dass** die Nadelanordnung (6) eine Kanüle (20), einen daran angeordneten Nadelträger (23) sowie ein den Nadelträger (23) aufnehmendes Führungselement (24) umfasst, wobei das Führungselement (24) mit einem dem distalen Ende (11) der Injektionsvorrichtung (1) zugewendeten Karpulenende (25) gekuppelt ist und in der Aufbewahrungsstellung das dem Karpulenende (25) zugewendete Nadelende (22) der Kanüle (20) einem durch das Nadelende (22) durchstechbaren Karpulenverschluss (26) vorgeordnet ist.

16. Injektionsvorrichtung nach Anspruch 15, **dadurch gekennzeichnet, dass** der Nadelträger (23) im Führungselement (24) axial verschiebbar gehalten ist und in der Aufbewahrungsstellung der Nadelträger (23) über zumindest ein lösbares Halteelement (63) relativ bezüglich dem Führungselement (24) in seiner Stellung daran festgelegt ist, oder dass am Halteelement (63) eine Stellnocke (64) angeordnet ist, welche das Halteelement (63) radial auf die von der Längsachse (13) abgewendete Seite überragt.

17. Injektionsvorrichtung nach einem der vorhergehenden Ansprüche, **dadurch gekennzeichnet, dass** eine Kolbenstange (67) der ersten Antriebsbaugruppe (4) eine radial umlaufende, als Vertiefung (94) ausgebildete Freistellung aufweist, welche in der Aufbewahrungsstellung der Injektionsvorrichtung (1) im Bereich des proximalen Endes der Karpule (3) angeordnet ist.

18. Injektionsvorrichtung nach einem der vorhergehenden Ansprüche, **dadurch gekennzeichnet, dass** nach Freigabe der am proximalen Ende (12) angeordneten Sicherungsvorrichtung (5) und bei einer nachfolgenden axialen Verstellung der Aktivierungshülse (9) relativ bezüglich des Traggehäuses (2) die erste Antriebsbaugruppe (4) freigegeben wird und die erste Antriebsbaugruppe (4) die Karpule (3) mitsamt der Nadelanordnung (6) hin in Richtung des distalen Endes (11) verlagert.

19. Injektionsvorrichtung nach einem der vorhergehenden Ansprüche, **dadurch gekennzeichnet, dass** das Traggehäuse (2), insbesondere der vordere Traggehäuseteil (27), an seinem distalen Ende (11) eine in Richtung auf die Längsachse (13) vorragende Steuerkurve (66) aufweist, welche nach einem vorbestimmbaren Verstellweg der Karpule (3) mit der am Halteelement (63) angeordneten Stellnocke (64) in Eingriff steht und dabei das lösbare Halteelement (63) des Nadelträgers (23) in eine Freigabestellung verstellt.

20. Injektionsvorrichtung nach einem der vorhergehenden Ansprüche, **dadurch gekennzeichnet, dass** der Nadelträger (23) in der Injektionsstellung am distalen Ende (11) des Traggehäuses (2) abgestützt ist und der Nadelträger (23) mitsamt der Kanüle (20) relativ bezüglich dem Führungselement (24) soweit in Richtung des mit dem durchstechbaren Karpulenverschluss (26) verschlossenen Karpulenendes (25) verlagert wird, dass das der Karpule (3) zugewendete Nadelende (22) der Kanüle (20) in Strömungsverbindung mit dem in der Karpule (3) enthaltenen Medikament (14) steht.

21. Injektionsvorrichtung nach einem der vorhergehenden Ansprüche, **dadurch gekennzeichnet, dass** mit der axialen Verstellung der Aktivierungshülse (9) relativ bezüglich des Traggehäuses (2) auch die zweite mit dem Nadelschutzelement (7) zusammenwirkende Antriebsbaugruppe (8) freigegeben wird und dabei das Nadelschutzelement (7) von seiner nicht wirksamen Stellung in eine das nach dem Injektionsvorgang über das distale Ende (11) des Traggehäuses (2) vorragende Nadelende (21) abdeckende Stellung verlagert wird.

22. Injektionsvorrichtung nach einem der vorhergehenden Ansprüche, **dadurch gekennzeichnet, dass** mit der axialen Verstellung der Aktivierungshülse (9) relativ bezüglich des Traggehäuses (2) die gleichzeitige Freigabe sowohl der ersten als auch der zweiten Antriebsbaugruppe (4, 8) erfolgt.

23. Injektionsvorrichtung nach einem der vorhergehenden Ansprüche, **dadurch gekennzeichnet, dass** die Sicherungsvorrichtung (5) eine Haltescheibe (17) umfasst, welche in ihrem Zentrum einen Durchbruch (72) sowie zusätzlich daran anschließende und sich radial nach außen erstreckende Ausnehmungen (73) für an der Haltescheibe (17) in der Aufbewahrungsstellung festzulegende Haltearme (16) der zweiten Antriebsbaugruppe (8) aufweist.

24. Injektionsvorrichtung nach einem der vorhergehenden Ansprüche, **dadurch gekennzeichnet, dass** vor dem Durchführen der Sterilisation derselben diese von einer bakteriendichten, jedoch für eine Begasung mittels Ethylenoxid gasdurchlässigen Umhüllung vollständig umschlossen ist.

25. Injektionsvorrichtung nach einem der vorhergehenden Ansprüche, **dadurch gekennzeichnet, dass** vor dem Durchführen der Sterilisation derselben diese in einer wannenförmigen Aufnahmeschale (75) aufgenommen ist und im Bereich eines Behälterrandes (78) die Aufnahmeschale (75) mittels einer bakteriendichten, jedoch für eine Begasung mittels Ethylenoxid gasdurchlässigen Abdeckung (79) verschlossen ist.

## Claims

1. Injection device (1), in particular an autoinjector, which is designed to be adjustable from
a storage position to an injection position, comprising
a support housing (2), which has a distal end (11) to be applied to a patient (10) and a proximal end (12) opposite the latter, between which a longitudinal axis (13) extends,
an activating sleeve (9), which surrounds the support housing (2) at least partly, whereby the distal end (11) of the support housing (2) projects over the activating sleeve (9) and the support housing (2) is surrounded by the activating sleeve (9) across the largest part of its longitudinal extension between the distal and the proximal end (11, 12), and the activating sleeve (9) can be displaced in axial direction relative to the support housing (2),
a cartridge (3) containing the medicine (14) to be administered in an injection procedure, whereby the cartridge (3) is mounted in the support housing (2),
a first drive unit (4) which is in active connection with the cartridge (3) and the activating sleeve (9) and can be triggered by means of the activating sleeve (9),
a safety device (5), which secures the first drive unit (4) prior to its activation by the activating sleeve (9) for the injection procedure in position relative to the support housing (2),
a needle arrangement (6), which is arranged ahead of the cartridge (3) in the storage position of the injection device (1) in the section of the distal end (11), and both needle ends (21, 22) of a cannula (20) are arranged inside the support housing (2),
a needle protection element (7) which can be displaced from a non-active position into a position which covers the needle end (21) projecting over the distal end (11) of the support housing (2) after the injection procedure,
a second activatable drive unit (8), which displaces the needle protection element (7) from the non-active position into the covering position,
**characterized in that**
the needle protection element (7) with the second drive unit (8) interacting therewith is arranged radially between the support housing (2) and the activating sleeve (9), as viewed in axial cross section and that the support housing (2) comprises a front and a rear support housing part (27, 28) with a front and rear holding chamber (29, 30) respectively, and the two support housing parts (27, 28) can be coupled together in a connecting section (31) via a coupling device (32) in the direction of the longitudinal axis (13) in two different longitudinal positions in a first and a second coupling position.

2. Injection device according to claim 1, **characterized in that** in the connecting section (31) of the coupling device (32) the front support housing part (27) grips over the rear support housing part (28).

3. Injection device according to one of claims 1 or 2, **characterized in that** the coupling device (32) on the two support housing parts (27, 28) comprises mutually cooperating locking elements (33, 34) and the latter are designed, so that the two support housing parts (27, 28) are locked respectively in the two coupling positions in an opposite direction of movement as viewed in the direction of the longitudinal axis (13) and moving away from each other.

4. Injection device according to one of claims 1 to 3, **characterized in that** the distal end (11) of the front support housing part (27) has an opening (35) allowing the passage of the needle end (21) of the needle arrangement (6), in which a penetrable sealing stopper (36) is arranged in a sealing position.

5. Injection device according to one of claims 1 to 4, **characterized in that** the front support housing part (27) in the connecting section (31) has a throughput (37).

6. Injection device according to one of claims 1 to 5, **characterized in that** the front support housing part (27) in the connecting section (31) on a face end wall (39) facing the rear support housing part (28) and springing back in the direction of the longitudinal axis (13) has a shoulder (41) designed to be wedge-shaped, as viewed in axial cross section, and to run continuously around the circumference.

7. Injection device according to one of claims 1 to 6, **characterized in that** on the rear support housing part (28) at one end (42) facing the front support housing part (27) a sealing element (43) designed to run continuously around the circumference is arranged.

8. Injection device according to claim 7, **characterized in that** the sealing element (43) bears in a sealing manner both against an external surface (44) of the cartridge (3) and an internal surface (45) of the front support housing part (27) and is arranged in the first coupling position of the two support housing parts (27, 28) spaced apart from the face end wall (39) or the shoulder (41) formed on the face end wall (39).

9. Injection device according to one of claims 1 to 8, **characterized in that** in the first coupling position of the two support housing parts (27, 28) the throughput (37) arranged in the front support housing part opens into the front holding chamber (29) of the front support housing part (27) and is in flow connection therewith.

10. Injection device according to one of claims 7 or 8, **characterized in that** in the second coupling position of the two support housing parts (27, 28) the sealing element (43) bears in a sealing manner against the backspringing face end wall (39) or the shoulder (41) arranged thereon, and also the access into the front holding chamber (29) is interrupted by the throughput (37).

11. Injection device according to one of claims 1 to 10, **characterized in that** the rear support housing part (28) on its external surface and in the region of its proximal end (12) has at least two detent noses (53, 54) spaced apart in the direction of the longitudinal axis (13), which interact with a detent element (55) arranged on the activating sleeve (9), such that the detent noses (53, 54) define the relative positions of the support housing (2) and the activating sleeve (9) relative to one another on the one hand in the storage position and on the other hand in the injection position, and **in that** only a relative longitudinal displacement of the activating sleeve (9) relative to the support housing (2) is permitted from the storage position to the injection position, but a relative longitudinal displacement in the opposite direction is prevented.

12. Injection device according to one of the preceding claims, **characterized in that** on the needle protection element (7) a rocker-like lever element (60) is arranged with lever ends (61, 62) spaced apart from one another in the direction of the longitudinal axis (13), whereby the lever element (60) in the storage position of the injection device (1) with its lever end (61) facing the distal end (11) of the support housing (2) is supported on a stop element (59) arranged on the support housing (2) on its external surface (58).

13. Injection device according to one of the preceding claims, **characterized in that** the activating sleeve (9) in the region of the distal end (11) and on its internal surface (56) comprises a stop surface (57), which is aligned preferably at right angles to the longitudinal axis (13), whereby the needle protection element (7) also comprises at its proximal end (12) an elastically deformable safety element (69), which in the position of the needle protection element (7) covering the needle arrangement (6) is supported on the stop surface (57) formed on the internal surface (56) of the activating sleeve (9).

14. Injection device according to one of the preceding claims, **characterized in that** the safety element (69) in the position of the needle protection element (7) covering the needle arrangement (6) is also supported radially on an adjusting element (70) arranged on the support housing (2), whereby the adjusting element (70) is designed to be continuous circumferentially and in the position of the needle protection element (7) covering the needle arrangement (6) the needle protection element (7) is supported with its proximal end (12) additionally on the adjusting element (70) in the direction of the distal end (11) of the support housing (2).

15. Injection device according to one of the preceding claims, **characterized in that** the needle arrangement (6) comprises a cannula (20), a needle support (23) arranged thereon and a guiding element (24) mounting the needle support (23), whereby the guiding element (24) is coupled with a cartridge end (25) facing the distal end (11) of the injection device (1) and in the storage position the needle end (22) of the cannula (20) facing the cartridge end (25) is arranged ahead of a cartridge seal (26) which can be penetrated by the needle end (22).

16. Injection device according to claim 15, **characterized in that** the needle support (23) is held axially displaceably in the guiding element (24) and is secured in its position thereon in the storage position of the needle support (23) by at least one releasable holding element (63) relative to the guiding element (24) or that an adjusting cam (64) is arranged on the holding element (63), which cam projects over the holding element (63) radially on the side opposite the longitudinal axis (13).

17. Injection device according to one of the preceding claims, **characterized in that** a piston rod (67) of the first drive unit (4) comprises a radially extending release designed as a depression (94) which is arranged in the storage position of the injection device (1) in the region of the proximal end of the cartridge (3).

18. Injection device according to one of the preceding claims, **characterized in that** after releasing the safety device (5) arranged at the proximal end (12) and with a subsequent axial displacement of the activating sleeve (9) relative to the support housing (2) the first drive unit (4) is released, and the first drive unit (4) displaces the cartridge (3) together with the needle arrangement (6) in the direction of the distal end (11).

19. Injection device according to one of the preceding claims, **characterized in that** the support housing (2), in particular the front support housing part (27), at its distal end (11) comprises a control cam (66) projecting in the direction of the longitudinal axis (13), which after a prespecifiable adjusting movement of the cartridge (3) is in engagement with the adjusting cam (64) arranged on the holding element (63), and thereby moves the detachable holding element (63) of the needle support (23) into a release position.

20. Injection device according to one of the preceding claims, **characterized in that** the needle support (23) is supported in the injection position on the distal end (11) of the support housing (2) and the needle support (23) together with the cannula (20) is displaced relative to the guiding element (24) so far in the direction of the cartridge end (25) closed by the penetrable cartridge seal (26), that the needle end (22) of the cannula (20) facing the cartridge (3) is in flow connection with the medicine (14) contained in the cartridge (3).

21. Injection device according to one of the preceding claims, **characterized in that** with the axial displacement of the activating sleeve (9) relative to the support housing (2) also the second drive unit (8) cooperating with the needle protection element (7) is released and thereby the needle protection element (7) is shifted from its non-effective position into a position covering the needle end (21) which projects over the distal end (11) of the support housing (2) after the injection procedure.

22. Injection device according to one of the preceding claims, **characterized in that** with the axial displacement of the activating sleeve (9) relative to the support housing (2) there is a simultaneous release of both the first and the second drive units (4, 8).

23. Injection device according to one of the preceding claims, **characterized in that** the safety device (5) comprises a holding disc (17), which in its centre comprises an opening (72) as well as additionally adjoining radially outwards extending recesses (73) for holding arms (16) of the second drive unit (8) to be secured to the holding disc (17) in the storage position.

24. Injection device according to one of the preceding claims, **characterized in that** prior to performing the sterilisation thereof the latter is surrounded completely by a bacteria-tight casing that is gas-permeable for gassing with ethylene oxide.

25. Injection device according to one of the preceding claims, **characterized in that** prior to performing the sterilisation thereof the latter is placed in a bath-like holding shell (75) and in the region of a container edge (78) the holding shell (75) is sealed by means of a bacteria-tight cover (79) that is gas-permeable for gassing with ethylene oxide.

## Revendications

1. Dispositif d'injection (1), plus particulièrement auto-injecteur, qui est conçu de manière réglable entre une position de stockage et une position d'injection, avec un boîtier de support (2), qui comprend une extrémité distale (11) pouvant être orientée vers un patient (10) et une extrémité proximale (12) opposée à celle-ci, entre lesquelles s'étend un axe longitudinal (13),
un fourreau d'activation (9), qui entoure au moins partiellement un boîtier de support (2), l'extrémité distale (11) du boîtier de support (2) dépassant du fourreau d'activation (9) et le boîtier de support (2) étant entouré, sur la majeure partie de son extension longitudinale entre les extrémités distale et proximale (11, 12), par le fourreau d'activation (9), le fourreau d'activation (9) étant réglable dans la direction axiale par rapport au boîtier de support (2).
une carpule (3) avec un médicament (14) contenu à l'intérieur et à administrer lors du processus d'injection, la carpule (3) étant logée dans le boîtier de support (2),
un premier sous-ensemble d'entraînement (4) qui est en liaison fonctionnelle avec la carpule (3) ainsi qu'avec le fourreau d'activation (9) et qui peut être déclenché à l'aide du fourreau d'activation (9),
un dispositif de fixation (5) qui maintient le premier sous-ensemble d'entraînement (4) en position jusqu'à son activation par le fourreau d'activation (9) pour le processus d'injection par rapport au boîtier de support (2),
un dispositif à aiguille (6) disposé, dans la position de stockage du dispositif d'injection (1), dans la section de l'extrémité distale (11) avant la carpule (3) et les deux extrémités de l'aiguille (21, 22) d'une canule (20) sont disposées à l'intérieur du boîtier de support (2), un élément de protection d'aiguille (7) qui peut être déplacé d'une position non active vers une position recouvrant l'extrémité d'aiguille (21) dépassant, après le processus d'injection, de l'extrémité distale (11) du boîtier de support (2),
un deuxième sous-ensemble d'entraînement (8) déclenchable qui déplace l'élément de protection d'aiguille (7) de la position non active vers la position de recouvrement,
**caractérisé en ce que**
l'élément de protection d'aiguille (7) est disposé, avec ce deuxième sous-ensemble d'entraînement (8) en interaction, radialement en vue en coupe axiale, entre le boîtier de support (2) et le fourreau d'activation (9) et **en ce que** le boîtier de support (2) comprend des parties avant et arrière du boîtier de support (27, 28) comprenant chacune un espace de logement avant et arrière (29, 30), et les deux parties du boîtier de support (27, 28) peuvent être couplées entre elles dans une section de liaison (31), à l'aide d'un dispositif d'accouplement (32), en direction de l'axe longitudinal (13), dans deux positions longitudinales différentes dans une première et une deuxième position d'accouplement.

2. Dispositif d'injection selon la revendication 1, **caractérisé en ce que**, dans la section de liaison (31) du dispositif d'accouplement (32), la partie avant du boîtier de support (27) passe par-dessus la partie de boîtier de support arrière (28).

3. Dispositif d'injection selon l'une des revendications 1 ou 2, **caractérisé en ce que** le dispositif d'accouplement (32) comprend, au niveau des deux parties du boîtier de support (27, 28), des éléments de blocage (33, 34) en interaction et ceux-ci sont conçus de façon à ce que les deux parties de boîtier de support (27, 28) sont bloquées chacune dans les deux positions d'accouplement dans une direction de mouvement à contre-sens ou d'éloignement, vue dans la direction de l'axe longitudinal (13).

4. Dispositif d'injection selon l'une des revendications 1 à 3, **caractérisé en ce que** l'extrémité distale (11) de la partie avant du boîtier de support (27) comprend une ouverture (35) permettant le passage de l'extrémité d'aiguille (21) du dispositif à aiguille (6), dans laquelle se trouve un obturateur étanche perçable (36) en position d'étanchéité.

5. Dispositif d'injection selon l'une des revendications 1 à 4, **caractérisé en ce que** la partie avant du boîtier de support (27) comprend, dans la section de liaison (31), un passage (37).

6. Dispositif d'injection selon l'une des revendications 1 à 5, **caractérisé en ce que** la partie avant du boîtier de support (27) comprend, dans la section de liaison (31), sur une paroi frontale (39) orientée vers la partie arrière du boîtier de support (28) et rentrant en direction de l'axe longitudinal (13), un épaulement (41) de forme conique en coupe axiale et réalisé de manière continue sur la circonférence.

7. Dispositif d'injection selon l'une des revendications 1 à 6, **caractérisé en ce que**, sur la partie arrière du boîtier de support (28), à une extrémité (42) orientée vers la partie avant du boîtier de support (27), se trouve un élément d'étanchéité (43) réalisé de manière continue sur la circonférence.

8. Dispositif d'injection selon la revendication 7, **caractérisé en ce que** l'élément d'étanchéité (43) s'appuie de manière étanche aussi bien contre une surface extérieur (44) de la carpule (3) que contre une surface intérieure (45) de la partie avant du boîtier de support (27) et, dans la première position d'accouplement des deux parties du boîtier de support (27, 28), éloigné de la paroi frontale (39) ou de l'épaulement (41) réalisé sur la paroi frontale (39).

9. Dispositif d'injection selon l'une des revendications 1 à 8, **caractérisé en ce que**, dans la première position d'accouplement des deux parties du boîtier de support (27, 28), le passage (37) disposé dans la partie avant du boîtier de support débouche dans l'espace de logement (29) de la partie avant du boîtier de support (27) et est en liaison d'écoulement avec celui-ci.

10. Dispositif d'injection selon l'une des revendications 7 ou 8, **caractérisé en ce que**, dans la deuxième position d'accouplement des deux parties du boîtier de support (27, 28), l'élément d'étanchéité (43) s'appuie de manière étanche contre la paroi frontale rentrante (39) ou contre l'épaulement (41) et, en outre, l'accès à l'espace de logement avant (29) est interrompu par le passage (37).

11. Dispositif d'injection selon l'une des revendications 1 à 10, **caractérisé en ce que** la partie arrière du boîtier de support (28) comprend, sur sa surface extérieure et au niveau de son extrémité proximale (12), au moins deux embouts d'encliquetage (53, 54) éloignés en direction de l'axe longitudinal (13), qui interagissent avec un élément d'encliquetage (55) disposé sur le fourreau d'activation (9) de façon à ce que les embouts d'encliquetage (53, 54) définissent les positions relatives du boîtier de support (2) et du fourreau d'activation (9) d'une part dans la position de stockage et d'autre part dans la position d'injection, et **en ce que** seul un réglage longitudinal relatif du fourreau d'activation (9) par rapport au boîtier de support (2), de la position de stockage vers la position d'injection soit possible, mais un réglage longitudinal relatif dans la direction opposé est empêché.

12. Dispositif d'injection selon l'une des revendications précédentes, **caractérisé en ce que**, sur l'élément de protection d'aiguille (7) se trouve un élément de levier (60) conçu comme une bascule, avec des extrémités de levier (61, 62) éloignés les uns des autres en direction de l'axe longitudinal (13), l'élément de levier (60) s'appuyant, dans la position de stockage du dispositif d'injection (1), avec son extrémité de levier (61) orientée vers l'extrémité distale (11) du boîtier de support (2), contre un élément de butée (59) disposé sur le boîtier de support (2), au niveau de sa surface extérieure (58).

13. Dispositif d'injection selon l'une des revendications précédentes, **caractérisé en ce que** le fourreau d'activation (9) comprend, au niveau de l'extrémité distale (11) ainsi qu'au niveau de sa surface intérieure (56), une surface de butée (57) qui est orientée de préférence dans la direction perpendiculaire à l'axe longitudinal (13), l'élément de protection d'aiguille (7) comprenant en outre, au niveau de son extrémité proximale (12) un élément de fixation (69) conçu de manière élastiquement déformable, qui s'appuie, dans la position de l'élément de protection d'aiguille (7) recouvrant le dispositif à aiguille (6), contre la surface intérieure (56) de la surface de butée (57) disposée sur la surface intérieure (56) du fourreau d'activation (9).

14. Dispositif d'injection selon l'une des revendications précédentes, **caractérisé en ce que** l'élément de fixation (69), dans la position de l'élément de protection d'aiguille (7) recouvrant le dispositif à aiguille (6), est en outre appuyé radialement contre un élément de réglage (70) disposé sur le boîtier de support (2), l'élément de réglage (70) étant conçu de manière continue sur la circonférence et, dans la position de l'élément de protection d'aiguille (7) recourant le dispositif à aiguille (6), l'élément de protection d'aiguille (7) est en outre appuyé, avec son extrémité proximale (12), contre l'élément de réglage (70) en direction de l'extrémité distale (11) du boîtier de support (2).

15. Dispositif d'injection selon l'une des revendications précédentes, **caractérisé en ce que** le dispositif à aiguille (6) comprend une canule (20), un support d'aiguille (23) disposé dessus ainsi qu'un élément de guidage (24) logeant le support d'aiguille (23), l'élément de guidage (24) étant couplé avec une extrémité de carpule (25) orientée vers l'extrémité distale (11) du dispositif d'injection (1) et, dans la position de stockage, l'extrémité d'aiguille (22) de la canule (20), orientée vers l'extrémité de la carpule (25) est disposée avant l'obturateur de carpule (26) perçable parl'extrémité d'aiguille (22).

16. Dispositif d'injection selon la revendication 15, **caractérisé en ce que** le support d'aiguille (23) est maintenu de manière coulissante axialement dans l'élément de guidage (24) et, dans la position de stockage, le support d'aiguille (23) est maintenu dans une position relative par rapport à l'élément de guidage (24) à l'aide d'au moins un élément de maintien (63) amovible, ou **en ce que**, sur l'élément de maintien (63), se trouve une came de réglage (64), qui dépasse de l'élément de maintien (63) radialement sur le côté opposé à l'axe longitudinal (13).

17. Dispositif d'injection selon l'une des revendications précédentes, **caractérisé en ce qu'**une tige de piston (67) du premier sous-ensemble d'entraînement (4) comprend un dégagement radial circulaire conçu comme une gorge (94), qui, dans la position de stockage du dispositif d'injection (1), est disposée au niveau de l'extrémité proximale de la carpule (3).

18. Dispositif d'injection selon l'une des revendications précédentes, **caractérisé en ce que**, après la libération du dispositif de fixation (5) disposé au niveau de l'extrémité proximale (12) et, lors d'un réglage axial suivant du fourreau d'activation (9) relativement par rapport au boîtier de support (2), le premier sous-ensemble d'entraînement (4) est libéré et le premier sous-ensemble d'entraînement (4) déplace la carpule (3) ainsi que le dispositif à aiguille (6) en direction de l'extrémité distale (11).

19. Dispositif d'injection selon l'une des revendications précédentes, **caractérisé en ce que** le boîtier de support (2), plus particulièrement la partie avant du boîtier de support (27), comprend, au niveau de son extrémité distale (11), une came de commande (66) dépassant en direction de l'axe longitudinal (13), qui, après un trajet de réglage prédéterminée de la carpule (3), se trouve en engrènement avec la came de réglage (64) disposée sur l'élément de maintien (63) et déplace alors l'élément de maintien (63) amovible du support d'aiguille (23) vers une position de libération.

20. Dispositif d'injection selon l'une des revendications précédentes, **caractérisé en ce que** le support d'aiguille (23) s'appuie, dans la position d'injection, contre l'extrémité distale (11) du boîtier de support (2) et le support d'aiguille (23) est déplacé, avec la canule (20), relativement par rapport à l'élément de guidage (24) dans la direction de l'extrémité de la carpule (25) obturée par l'obturateur de carpule (26) perçable, jusqu'à ce que l'extrémité d'aiguille (22) de la canule (20) orientée vers la carpule (3) soit en liaison d'écoulement avec le médicament (14) contenu dans la carpule (3).

21. Dispositif d'injection selon l'une des revendications précédentes, **caractérisé en ce que**, avec le réglage axial du fourreau d'activation (9) relativement par rapport au boîtier de support (2), le deuxième sous-ensemble d'entraînement (8), qui interagit avec l'élément de protection d'aiguille (7), est également libéré et l'élément de protection d'aiguille (7) est déplacé de sa position efficace vers une position recouvrant l'extrémité de l'aiguille (21) dépassant de l'extrémité distale (11) du boîtier de support (2) après le processus d'injection.

22. Dispositif d'injection selon l'une des revendications précédentes, **caractérisé en ce que**, avec le réglage axial du fourreau d'activation (9) relativement par rapport au boîtier de support (2), a lieu la libération simultanée du premier ainsi que du deuxième sous-ensemble d'entraînement (4, 8).

23. Dispositif d'injection selon l'une des revendications précédentes, **caractérisé en ce que** le dispositif de fixation (5) comprend une rondelle de maintien (17) qui présente, en son centre, un passage (72) ainsi que des évidements (73) reliés à celui-ci et s'étendant radialement vers l'extérieur pour des bras de maintien (16) du deuxième sous-ensemble d'entraînement (8) à fixer sur la rondelle de maintien (17) dans la position de stockage.

24. Dispositif d'injection selon l'une des revendications précédentes, **caractérisé en ce que**, avant la réalisation de la stérilisation de celui-ci, elle est entièrement entourée d'une enveloppe étanche aux bactéries mais perméable aux gaz pour un gazage à l'aide d'oxyde d'éthylène.

25. Dispositif d'injection selon l'une des revendications précédentes, **caractérisé en ce que**, avant la réalisation de la stérilisation de celui-ci, elle est logée dans une coque de logement (75) en forme de bac et, au niveau d'un bord du récipient (78), la coque de logement (75) est fermée à l'aide d'un couvercle (79) étanche aux bactéries mais perméable aux gaz pour un gazage à l'oxyde d'éthylène.
